# EUROPEAN PATENT APPLICATION

(11) **EP 4 286 510 A1**
(43) Date of publication of application: **06.12.2023**
(21) Application number: 22745867.6
(22) Date of filing: 25.01.2022
(51) Int. Cl.: C12N 1/21, A61K 35/745, A61K 39/215, A61P 31/14, A61P 37/04, C07K 14/165, C07K 14/195, C07K 19/00, C12N 15/31, C12N 15/50, C12N 15/62

(54) **ORAL CORONAVIRUS INFECTION VACCINE**

(30) Priority: 26.01.2021 JP 2021010009
(71) Applicant: National University Corporation Kobe University, Kobe-shi, Hyogo 657-8501 (JP); Kyoto University, Kyoto-shi, Kyoto 606-8501 (JP)
(72) Inventor: SHIRAKAWA, Toshiro, Kobe-shi, Hyogo 657-8501 (JP); UENO, Shunpei, Kobe-shi, Hyogo 657-8501 (JP); KITAGAWA, Koichi, Kobe-shi, Hyogo 657-8501 (JP); KONDO, Akihiko, Kobe-shi, Hyogo 657-8501 (JP); KAMEOKA, Masanori, Kobe-shi, Hyogo 657-8501 (JP); KATAYAMA, Takane, Kyoto-shi, Kyoto 606-8501 (JP)
(74) Representative: Arnold & Siedsma
(86) International application number: PCT/JP2022/002677
(87) International publication number: WO 2022/163647

(57) **Abstract**

Provided is an orally administrable vaccine against a coronavirus infectious disease. A transformed *Bifidobacterium* designed to display a part or a whole of a constituent protein of a coronavirus on a surface of the *Bifidobacterium* enables the provision of the orally administrable vaccine against a coronavirus infectious disease. The transformed *Bifidobacterium* designed to display a part or a whole of a constituent protein of a coronavirus on a surface of the *Bifidobacterium* can induce humoral immunity and cellular immunity through oral administration to suppress an increase in severity of pneumonia or the like even after viral infection.

## Description

### Technical Field

The present invention relates to a transformed *Bifidobacterium* designed to display a part or a whole of a constituent protein of a coronavirus on a surface of the *Bifidobacterium.* The present invention also relates to an orally administrable coronavirus infectious disease vaccine containing the transformed *Bifidobacterium* as an active ingredient.

The present application claims priority from Japanese Patent Application No. 2021-010009, which is incorporated herein by reference.

### Background Art

A coronavirus is an RNA virus belonging to the order *Nidovirales,* and is known to be a causal virus of Middle East respiratory syndrome (MERS) or severe acute respiratory syndrome (SARS), or a causal virus of common cold (cold). The coronavirus has a diameter of from about 100 nm to about 160 nm, and has the largest genetic substance of all RNA viruses. A spike protein (S), an envelope glycoprotein (E: envelope protein), a membrane glycoprotein (M: membrane protein), and the like are present on a surface of a lipid membrane of a virion. A new kind of coronavirus was detected from a patient with pneumonia of unknown cause that occurred in December 2019, and this novel coronavirus was named "severe acute respiratory syndrome coronavirus 2: SARS-CoV-2." On February 11, 2020, the World Health Organization (WHO) officially named the novel coronavirus infectious disease "coronavirus disease 2019 (COVID-19)."

Numerous research and development efforts on vaccines for COVID-19 are being vigorously conducted in Japan and abroad with an aim of achieving practical application early. According to a WHO summary as of December 16, 2020, there are about 56 kinds of COVID-19 candidate vaccines in clinical trials, and there are another 166 kinds in preclinical phases. Development of various kinds of vaccines, such as an inactivated vaccine, a recombinant protein vaccine, a peptide vaccine, a messenger RNA (mRNA) vaccine, a DNA vaccine, and a viral vector vaccine, is being conducted. The mRNA vaccine requires ultra-low temperature control at -70°C or less in order to be stored, and also has problems with a storage method, a transportation method, and the like. It is conceived that the COVID-19 vaccines currently under development for practical application still have many problems left in terms of administration method, storage control method, and the like as well as effect and safety.

A cell membrane of a cell is a biological membrane that separates the inside of the cell from the outside. On a surface of the cell membrane, there are a large number of membrane proteins each having a function of providing information on the cell or a function of transporting a substance endogenous or exogenous to the cell. The following concept has been proposed: a certain antigen is fused to a membrane protein so as to be displayed on a cell surface of a microorganism and be used as an oral vaccine for artificially inducing antigen-specific acquired immunity. For example, there is known an example in which a vector having a gene encoding a membrane-binding portion of an enzyme protein such as poly-γ-glutamate synthetase is utilized to display a target protein on a cell surface of a host microorganism (Patent Literature 1). In addition, with regard to a technology involving using, as a vaccine, a flagellin protein derived from a bacterium that causes an infectious disease, there is a report on an oral vaccine containing, as a capsule content, a transformed microorganism expressing flagellin (Patent Literature 2). In Patent Literature 2, it is reported that the transformed microorganism is prepared using, as the bacterium to be caused to produce flagellin, any of intestinal bacteria that are commonly referred to as good bacteria, such as microorganisms belonging to the genus *Bifidobacterium* (which are collectively referred to as *"Bifidobacterium"*) or lactic acid bacteria.

The *Bifidobacterium* is an indigenous bacterium found downstream in the small intestine of a human or other animals, or in the large intestine thereof. The *Bifidobacterium* is an obligately anaerobic Gram-positive bacterium, and hence has high selectivity in culture. Besides, the *Bifidobacterium* has high biocompatibility and does not have endotoxins, which are found in Gram-negative bacteria, and hence the *Bifidobacterium* is highly safe. Accordingly, the *Bifidobacterium* has been GRAS (Generally Recognized As Safe)-approved according to a standard of a review system regarding food safety. In addition, there is a report that the *Bifidobacterium* has a property of binding to mucus formed of mucin with which the intestinal tract is covered. Accordingly, the *Bifidobacterium* is considered to have a higher property of adhering to the intestinal wall than those of other bacteria in the intestines. There have already been developed and reported a technology for expressing and displaying a protein or a peptide on a surface of such *Bifidobacterium,* and a technology concerning a novel vaccine based on the *Bifidobacterium,* which uses the above-mentioned technology (Patent Literatures 3, 4, and 5).

### Citation List

### Patent Literature

[PTL 1] WO 03/014360 A1 (JP 2005-500054 A)
[PTL 2] WO 2008/114889 A1 (JP 5187642 B2)
[PTL 3] WO 2011/034181 A1 (JP 5561681 B2)
[PTL 4] WO 2016/208332 A1 (JP 6770269 B2)
[PTL 5] WO 2018/123507 A1 (JP 6810877 B2)

### Summary of Invention

### Technical Problem

The present invention provides an orally administrable vaccine against a coronavirus infectious disease.

### Solution to Problem

The inventors of the present invention have made extensive investigations in order to solve the above-mentioned problems, and as a result, have found that a transformed *Bifidobacterium* designed to display a part or a whole of a constituent protein of a coronavirus on a surface of the *Bifidobacterium* can induce humoral immunity and cellular immunity through oral administration. Thus, the inventors have completed the present invention.

That is, the present invention includes the following.
1. A transformed *Bifidobacterium,* which is designed to display a part or a whole of a constituent protein of a coronavirus as a coronavirus antigen on a surface of the *Bifidobacterium.*
2. The transformed *Bifidobacterium* according to the above-mentioned item 1, wherein the part of the constituent protein of the coronavirus is a part or a whole of an S protein of the coronavirus.
3. The transformed *Bifidobacterium* according to the above-mentioned item 1 or 2, wherein the transformed *Bifidobacterium* designed to display on the surface of the *Bifidobacterium* includes: DNA encoding the part or the whole of the constituent protein of the coronavirus; and DNA encoding a membrane protein derived from a bacterium.
4. The transformed *Bifidobacterium* according to the above-mentioned item 3, wherein the membrane protein derived from a bacterium is a GNB/LNB substrate-binding membrane protein derived from a *Bifidobacterium.*
5. The transformed *Bifidobacterium* according to any one of the above-mentioned items 1 to 4, wherein the coronavirus is SARS-COV-2.
6. The transformed *Bifidobacterium* according to the above-mentioned item 5, wherein the S protein of SARS-COV-2 is any one of the following items 1) to 5):
   1) a protein identified by an amino acid sequence set forth in SEQ ID NO: 1;
   2) a protein identified by an amino acid sequence having one or a plurality of amino acids substituted, deleted, added, or introduced in the amino acid sequence set forth in SEQ ID NO: 1;
   3) a protein identified by amino acids of the amino acid sequence set forth in SEQ ID NO: 1 in which amino acid 501 is substituted from asparagine (N) to tyrosine (Y);
   4) a protein identified by amino acids of the amino acid sequence set forth in SEQ ID NO: 1 in which amino acid 614 is substituted from aspartic acid (D) to glycine (G); and
   5) a protein identified by an amino acid sequence having 60% or more homology to the amino acid sequence set forth in SEQ ID NO: 1.
7. The transformed *Bifidobacterium* according to the above-mentioned item 5 or 6, wherein the part of the S protein of SARS-COV-2 contains an S1 protein.
8. The transformed *Bifidobacterium* according to the above-mentioned item 7, wherein the S1 protein of SARS-COV-2 contains an S1B protein portion of SARS-COV-2.
9. The transformed *Bifidobacterium* according to the above-mentioned item 8, wherein the S1B protein of SARS-COV-2 is any one of the following items 1) to 4):
   1) a protein containing at least an amino acid sequence (SEQ ID NO: 2) from position 332 to position 526 of an amino acid sequence set forth in SEQ ID NO: 1;
   2) a protein containing an amino acid sequence having one or a plurality of amino acids substituted, deleted, added, or introduced in the amino acid sequence set forth in SEQ ID NO: 2, the protein having immunogenicity for production of an anti-SARS-COV-2 antibody;
   3) a protein identified by amino acids of at least the amino acid sequence from position 332 to position 526 of the amino acid sequence set forth in SEQ ID NO: 1 in which amino acid 501 with reference to the amino acid sequence set forth in SEQ ID NO: 1 is substituted from asparagine (N) to tyrosine (Y); and
   4) a protein identified by an amino acid sequence having 60% or more homology to the amino acid sequence set forth in SEQ ID NO: 2, the protein having immunogenicity for production of an anti-coronavirus antibody.
10. The transformed *Bifidobacterium* according to any one of the above-mentioned items 4 to 9, wherein the transformed *Bifidobacterium* includes DNA encoding a protein having an adjuvant function between DNA encoding a part or a whole of an S protein of the coronavirus and DNA encoding a GNB/LNB substrate-binding membrane protein derived from a *Bifidobacterium.*
11. The transformed *Bifidobacterium* according to any one of the above-mentioned items 1 to 10, wherein the coronavirus antigen to be displayed on the surface of the *Bifidobacterium* is a fusion protein of: a protein containing a part or a whole of an S protein of the coronavirus; and a GNB/LNB substrate-binding membrane protein derived from a *Bifidobacterium.*
12. A coronavirus infectious disease vaccine formulation, including the transformed *Bifidobacterium* of any one of the above-mentioned items 1 to 11 as an active ingredient of a coronavirus infectious disease vaccine.
13. The coronavirus infectious disease vaccine formulation according to the above-mentioned item 12, wherein the coronavirus infectious disease vaccine formulation is an oral formulation.
14. A coronavirus infectious disease vaccine formulation, including as an active ingredient a protein to be displayed on a surface of a *Bifidobacterium,* the protein being produced from the transformed *Bifidobacterium* of any one of the above-mentioned items 1 to 11 and being a part or a whole of a constituent protein of a coronavirus.
15. A method of preventing coronaviral infection and/or a method of preventing an increase in severity after coronaviral infection, including administering the coronavirus infectious disease vaccine formulation of any one of the above-mentioned items 12 to 14.
16. A method of preventing and/or treating a sequela after coronaviral infection, including administering the coronavirus infectious disease vaccine formulation of any one of the above-mentioned items 12 to 14.
17. A method of boosting immunity against a coronavirus, including administering the coronavirus infectious disease vaccine formulation of any one of the above-mentioned items 12 to 14.
18. The method of boosting immunity according to the above-mentioned item 17, wherein the boosting immunity is boosting of humoral immunity and/or cellular immunity.
19. A method of administering a coronavirus infectious disease vaccine formulation, including administering the coronavirus infectious disease vaccine formulation of any one of the above-mentioned items 12 to 14 with an adjuvant.
20. A method of administering a coronavirus infectious disease vaccine formulation, including administering the coronavirus infectious disease vaccine formulation of any one of the above-mentioned items 12 to 14 by oral administration.
21. A method of generating a coronavirus infectious disease vaccine formulation, including a step of performing design so that a part or a whole of a constituent protein of a coronavirus is displayed as a coronavirus antigen on a surface of a *Bifidobacterium.*

### Advantageous Effects of Invention

According to the transformed *Bifidobacterium* designed to display a part or a whole of a constituent protein of a coronavirus on a surface of the *Bifidobacterium* of the present invention, humoral immunity and cellular immunity can be induced through oral administration.

### Brief Description of Drawings

FIG. 1 is a diagram for illustrating the concepts of: shuttle vectors having DNAs respectively encoding an S1 protein (S1: 1-681), an S1AB protein (S1AB: 1-583), and an S1B protein 1 (S1B: 330-583) and an S1B protein 2 (S1B: 332-526), out of partial proteins of the S protein of SARS-COV-2, downstream of a GLBP gene; and a fusion protein of a GLBP and a partial protein of the S protein of the coronavirus expressed on the surface of a *Bifidobacterium.* (Examples 1 and 2)
FIG. 2 is a diagram for illustrating a *Bifidobacterium-E. coli* shuttle vector (pHY69-1). (Example 1)
FIG. 3 is a diagram for illustrating the concept of a pHY plasmid carrying a gene encoding the partial protein (S1B) of the S protein of SARS-COV-2. (Example 1)
FIG. 4 is a diagram for illustrating a method of producing a *Bifidobacterium-E. coli* shuttle vector (pMNO101) for the generation of a transformed *Bifidobacterium* for the expression of the fusion protein of the GLBP and S1B: 332-526 (Example 2).
FIG. 5-1 shows a part (first half) of the base sequence (SEQ ID NO: 19) of a GLBP-S1 B-332-526 fusion gene containing a promoter region introduced into a *Bifidobacterium-E. coli* shuttle vector pJW241. (Example 2)
FIG. 5-2 shows a part (second half) of the base sequence (SEQ ID NO: 19) of the GLBP-S1B-332-526 fusion gene containing a promoter region introduced into the *Bifidobacterium-E. coli* shuttle vector pJW241. (Example 2)
FIG. 6 shows the amino acid sequence (SEQ ID NO: 20) of a GLBP-S1B-332-526 fusion protein containing a promoter region introduced into the *Bifidobacterium-E. coli* shuttle vector pJW241. (Example 2)
FIG. 7-1 shows a part (first half) of the base sequence (SEQ ID NO: 21) of a GLBP-S1B-332-526 N501Y fusion gene containing a promoter region introduced into the *Bifidobacterium-E. coli* shuttle vector pJW241. (Example 3)
FIG. 7-2 shows a part (second half) of the base sequence (SEQ ID NO: 21) of the GLBP-S1B-332-526 N501Y fusion gene containing a promoter region introduced into the *Bifidobacterium-E. coli* shuttle vector pJW241. (Example 3)
FIG. 8 shows the amino acid sequence (SEQ ID NO: 22) of a GLBP-S1B-332-526 N501Y fusion protein containing a promoter region introduced into the *Bifidobacterium-E. coli* shuttle vector pJW241. (Example 3)
FIG. 9 is an image showing results of determination of surface expression of each fusion protein of the GLBP and a partial protein of the S protein of SARS-COV-2 in a transformed *Bifidobacterium* of the present invention by western blotting using an anti-S1B-RBD protein IgG antibody. PHY represents the result of a transformed *Bifidobacterium* using a plasmid containing no gene encoding a partial protein of the S protein of SARS-COV-2. (Example 4)
FIG. 10 includes images showing results of determination of surface expression of a fusion protein of the GLBP and S1B-332-526 in the transformed *Bifidobacterium* of the present invention by western blotting using an anti-GLBP antibody. (Example 4)
FIG. 11 is an image showing results of determination of surface expression of a fusion protein of the GLBP and S1B-330-583 in the transformed *Bifidobacterium* of the present invention by western blotting using an anti-GLBP antibody. (Example 4)
FIG. 12 includes charts showing results of determination by flow cytometry of surface expression of each of the fusion protein of the GLBP and S1B-332-526 and the fusion protein of the GLBP and S1B-330-583 in the transformed *Bifidobacterium* of the present invention. (Example 5)
FIG. 13 is a graph showing results of determination by flow cytometry of surface expression of each of the fusion protein of the GLBP and S1B-332-526 and the fusion protein of the GLBP and S1B-330-583 in the transformed *Bifidobacterium* of the present invention. (Example 5)
FIG. 14 is a diagram for illustrating an experimental protocol for determining an immunity-inducing effect achieved when the transformed *Bifidobacterium* of the present invention was orally administered to mice. (Examples 6 and 7)
FIG. 15 is a graph showing results of determination by an ELISA method of an ability to induce an anti-S1B-RBD protein IgG antibody in mouse serum when the transformed *Bifidobacterium* of the present invention (B-COV-S1B-330-583) was orally administered to mice. PHY represents results obtained when a transformed *Bifidobacterium* with a plasmid containing no gene encoding a partial protein of the S protein of SARS-COV-2 was orally administered. (Example 6)
FIG. 16 includes graphs showing results of determination by an ELISA method of induction of an anti-S1B-RBD protein IgG antibody in mouse serum achieved when the transformed *Bifidobacterium* of the present invention (B-COV-S1 B-330-583 or B-COV-S1B-332-526) was orally administered to mice. PHY represents results obtained when the transformed *Bifidobacterium* with a plasmid containing no gene encoding a partial protein of the S protein of SARS-COV-2 was orally administered. (Example 6)
FIG. 17 includes graphs showing results of recognition by an ELISA method of induction of an anti-S1B-RBD protein IgM antibody in mouse serum achieved when the transformed *Bifidobacterium* of the present invention (B-COV-S1B-330-583 or B-COV-S1B-332-526) was orally administered to mice. PHY represents results obtained when the transformed *Bifidobacterium* with a plasmid containing no gene encoding a partial protein of the S protein of SARS-COV-2 was orally administered. (Example 6)
FIG. 18 includes graphs showing results of determination with a peptide MBL037 of an S1B protein-specific cellular immune response-inducing effect in mouse spleen cells achieved when the transformed *Bifidobacterium* of the present invention (B-COV-S1B-330-583 or B-COV-S1B-332-526) was orally administered to mice. PHY represents results obtained when the transformed *Bifidobacterium* with a plasmid containing no gene encoding a partial protein of the S protein of SARS-COV-2 was orally administered. (Example 7)
FIG. 19 includes graphs showing results of determination with a peptide MBL045 of an S1B protein-specific cellular immune response-inducing effect in mouse spleen cells achieved when the transformed *Bifidobacterium* of the present invention (B-COV-S1B-330-583 or B-COV-S1B-332-526) was orally administered to mice. PHY represents results obtained when the transformed *Bifidobacterium* with a plasmid containing no gene encoding a partial protein of the S protein of SARS-COV-2 was orally administered. (Example 7)
FIGS. 20 are a diagram and graphs showing results of determination with the peptide MBL045 of an S1B protein-specific cellular immune response-inducing effect in mouse spleen cells achieved when the transformed *Bifidobacterium* of the present invention (B-COV-S1B-332-526) was orally administered to mice. FIG. 20A is an illustration of an administration protocol, FIG. 20B shows CD4⁺ induction results, and FIG. 20C shows CD8⁺ induction results. (Example 8)
FIGS. 21 are a diagram and a graph showing results of determination of a humoral immune response-inducing effect in mouse lung tissue achieved when the transformed *Bifidobacterium* of the present invention (B-COV-S1B-332-526) was orally administered to mice. FIG. 21A is an illustration of an administration protocol, and FIG. 21B shows IgA induction results. (Example 9)
FIGS. 22 show IgG antibody induction results obtained when the transformed *Bifidobacterium* of the present invention (B-COV-S1B-332-526) was orally administered to mice together with various adjuvants. FIG. 22A is an illustration of an administration protocol, and FIG. 22B shows IgG antibody induction results. (Example 10)
FIG. 23-1 is a diagram for illustrating a vector for generating a transformed *Bifidobacterium* B-COV-S1B-332-526 N501Y. (Example 11)
FIG. 23-2 is a diagram for illustrating a vector for generating a transformed *Bifidobacterium* B-COV-S1B-332-526 N501Y-CD40L. (Example 11)
FIGS. 24 are a diagram and graphs showing results of determination of an IgG antibody induction ability and an IgA antibody induction ability exhibited when the transformed *Bifidobacterium* of the present invention (B-COV-S1B-332-526 N501Y or B-COV-S1B-332-526 N501Y-CD40L) was orally administered to mice. FIG. 24A is an illustration of an administration protocol, FIG. 24B shows IgG antibody induction results, and FIG. 24C shows IgA antibody induction results. (Example 12)
FIGS. 25 are a diagram and graphs showing results of a SARS-CoV-2 viral infection experiment on hamsters. FIG. 25A is an illustration of a protocol for administration of the transformed *Bifidobacterium* of the present invention (B-COV-S1B-332-526 N501Y) and viral infection (Examples 13 and 14), and FIG. 25B shows viral loads in lung tissue of hamsters virally infected after oral administration of the transformed *Bifidobacterium* (B-COV-S1B-332-526 N501Y-CD40L). (Example 13)
FIGS. 26 are graphs showing results of a SARS-CoV-2 viral infection experiment on hamsters. FIG. 26A shows body weight changes of hamsters virally infected after oral administration of the transformed *Bifidobacterium* (B-COV-S1B-332-526 N501Y-CD40L), and FIG. 26B shows viral loads in lung tissue after viral infection. (Example 14)
FIG. 27 includes pictures showing results of the SARS-CoV-2 viral infection experiment on hamsters. Lung finding results of hamsters virally infected after oral administration of the transformed *Bifidobacterium* (B-COV-S1B-332-526 N501Y-CD40L) are shown. (Example 14)
FIGS. 28 are images and a graph showing results of the SARS-CoV-2 viral infection experiment on hamsters. Lung tissue finding results of hamsters virally infected after oral administration of the transformed *Bifidobacterium* (B-COV-S1B-332-526 N501Y-CD40L) are shown. (Example 14)

### Description of Embodiments

The present invention relates to a transformed *Bifidobacterium* designed to display a part or a whole of a constituent protein of a coronavirus on a surface of the *Bifidobacterium.* The present invention also relates to an orally administrable coronavirus infectious disease vaccine containing the transformed *Bifidobacterium* as an active ingredient.

### (Coronavirus)

The coronavirus is classified into the family *Coronaviridae* of the suborder *Cornidovirineae* of the order *Nidovirales.* The family *Coronaviridae* is further classified into the subfamily *Letovirinae* and the subfamily *Orthocoronavirinae.* As a virus identified as a human pathogenic coronavirus, there are given, for example, so-called common cold coronaviruses (e.g., human coronavirus 229E, human coronavirus NL63, human coronavirus OC43, and human coronavirus HKU1), and causal viruses of severe acute respiratory syndrome (SARS), Middle East respiratory syndrome (MERS), and the like. The coronavirus of the present invention only needs to be a virus classified as a human or animal pathogenic coronavirus, and is not particularly limited, but the present invention suitably relates to the so-called novel coronavirus, which was named SARS-CoV-2 in 2020.

The coronavirus is an RNA virus enclosed in an envelope. The protein of the coronavirus includes a structural protein and a nonstructural protein. The structural protein includes, for example, a spike glycoprotein (S protein), a small envelope glycoprotein (E protein), and a membrane glycoprotein (M protein) each present on a surface of a lipid membrane of a virion, and an N protein for forming a nucleocapsid inside the virion. The nonstructural protein includes proteins having functions, such as protease, primase, RNA polymerase, helicase, nuclease, ribonuclease, and methyltransferase. Binding between the S protein of the virus and a host cell receptor mainly mediates viral invasion, and also determines the tissue or host preference of the virus. The S protein of the coronavirus contains an S1 receptor binding domain (receptor binding domain, hereinafter sometimes referred to simply as "RBD") and a second domain S2, which mediates membrane fusion between the virus and a host cell. The S1 receptor binding domain is further classified into A and B.

The "part or whole of a constituent protein of a coronavirus" serving as a coronavirus antigen in the present invention may be the whole of any of the above-mentioned structural proteins or nonstructural proteins, or may be a part thereof, but the S protein is suitable as a protein to be used as a part. Further, a part or the whole of the S protein may be used, and a part of the S1 receptor binding domain (RBD) is particularly suitable. The S protein of SARS-CoV-2 is exemplified by a protein containing an amino acid sequence having 1,273 amino acids set forth in GenBank ACCESSION: BCB97891.1 (SEQ ID NO: 1).

· SARS-CoV-2 (S protein) GenBank ACCESSION: BCB97891.1 (SEQ ID NO: 1)

Genome replication of an RNA virus is governed by an RNA-dependent RNA polymerase (RdRp), which carries out RNA synthesis with RNA as a template. This enzyme does not have a proofreading function for correcting replication errors, and hence results in a high mutation frequency. The coronavirus is a virus that is extremely prone to mutations at the time of viral replication. Many mutations do not affect the function of the virus, but a mutation at an important site in the S protein, which is conceived to determine reactivity with an antibody, is predicted to significantly change the structure of the protein. For example, N501Y-type and D614G-type mutant viruses and the like account for the majority of SARS-CoV-2 in Europe and the like. Further, there is also a fear of the occurrence of a new mutant virus. For example, there is a fear in that novel SARS-CoV-2 variants VOC-202012/01 and 501Y.V2 may have increased infectivity. VOC-202012/01 has 23 mutations, and is defined by mutations in the S protein (deletion 69-70, deletion 144, N501Y, A570D, D614G, P681H, T716I, S982A, and D1118H) and mutations at other sites (Public Health England. Investigation of novel SARS-COV-2 variant: Variant of Concern 202012/01. December 21, 2020.). The SARS-COV-2 that was named "the Omicron variant" in November 2021 has the following features as compared to a reference strain: having about 30 amino acid substitution-causing mutations in the spike protein; and having three small deletions and one insertion site. Of those, about 15 mutations are present in the receptor binding domain (receptor binding protein (RBD); residues 319-541). Mutations and deletions recognized in 50% or more of 525 Omicron variants registered on Global Initiative on Sharing All Influenza Data (international genome database: GISAID, https://www.gisaid.org) as of December 6, 2021 are as follows: A67V, del60/70, T95I, G142D, del143/145, N21I, del212/212, G339D, S371L, S373P, S375F, S477N, T478K, E484A, Q493R, G496S, Q498R, N501Y, Y505H, T547K, D614G, H655Y, N679K, P681H, N764K, D796Y, N856K, Q954H, N969K, and L981F (from the website of National Institute of Infectious Diseases: December 8, 2021). In view of the foregoing, it is conceived that, of the constituent proteins of the coronavirus as used herein, in particular, the S protein should not be limited to the amino acid sequence identified in GenBank ACCESSION: BCB97891.1.

The S protein of the coronavirus as used herein may be identified by any one of the following items 1) to 5):
1) a protein identified by an amino acid sequence set forth in SEQ ID NO: 1;
2) a protein identified by an amino acid sequence having one or a plurality of amino acids substituted, deleted, added, or introduced in the amino acid sequence set forth in SEQ ID NO: 1;
3) a protein identified by amino acids of the amino acid sequence set forth in SEQ ID NO: 1 in which amino acid 501 is substituted from asparagine (N) to tyrosine (Y);
4) a protein identified by amino acids of the amino acid sequence set forth in SEQ ID NO: 1 in which amino acid 614 is substituted from aspartic acid (D) to glycine (G); and
5) a protein identified by an amino acid sequence having 60% or more homology to the amino acid sequence set forth in SEQ ID NO: 1.

The part of the S protein of the coronavirus to be used in the present invention is particularly suitably a part contained in an S1 protein out of the S proteins of the coronavirus, the part having antigenicity.

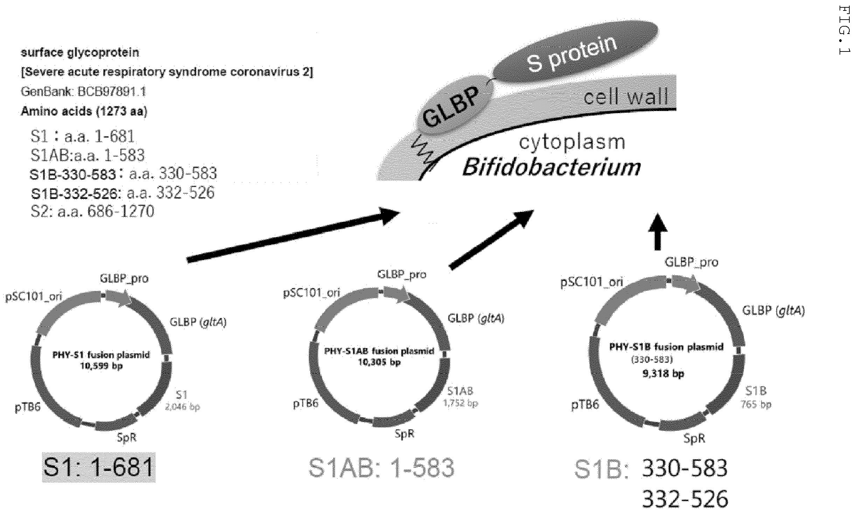

In particular, the S1 protein of SARS-COV-2 to be used in the present invention contains an S1B protein. Specifically, the S1 protein of SARS-COV-2 refers to a protein containing at least an amino acid sequence from position 332 to position 526 (SEQ ID NO: 2) with reference to the amino acid sequence set forth in SEQ ID NO: 1. More specific examples thereof include the following, with reference to the amino acid sequence set forth in SEQ ID NO: 1: an S1 protein (S1: 1-681) identified by an amino acid sequence shown at position 1 to position 681; an S1AB protein (S1AB: 1-583) identified by an amino acid sequence shown at position 1 to position 583; an S1B protein 1 (S1B: 330-583) identified by an amino acid sequence shown at position 330 to position 583; and an S1B protein 2 (S1B: 332-526) identified by the amino acid sequence shown at position 332 to position 526 (SEQ ID NO: 2) (see FIG. 1). Further, in consideration of the fact that the coronavirus has the property of being prone to mutations, one or a plurality of amino acids may be substituted, deleted, added, or introduced with respect to the above-mentioned identifying amino acids.

· Amino acid sequence from position 332 to position 526 of amino acid sequence identified in GenBank ACCESSION: BCB97891.1 (SEQ ID NO: 2)

itnlcpfge vfnatrfasv yawnrkrisn cvadysvlyn sasfstfkcy gvsptklndl cftnvyadsf virgdevrqi apgqtgkiad

ynyklpddft gcviawnsnn ldskvggnyn ylyrlfrksn lkpferdist eiyqagstpc ngvegfncyf plqsygfqpt ngvgyqpyrv

vvlsfellha patvcg

The S1B protein of SARS-COV-2 to be used in the present invention may be identified by any one of the following items 1) to 4):
1) a protein containing at least an amino acid sequence (SEQ ID NO: 2) from position 332 to position 526 of an amino acid sequence set forth in SEQ ID NO: 1;
2) a protein containing an amino acid sequence having one or a plurality of amino acids substituted, deleted, added, or introduced in the amino acid sequence set forth in SEQ ID NO: 2, the protein having immunogenicity for production of an anti-SARS-COV-2 antibody;
3) a protein identified by amino acids of at least the amino acid sequence from position 332 to position 526 of the amino acid sequence set forth in SEQ ID NO: 1 in which amino acid 501 with reference to the amino acid sequence set forth in SEQ ID NO: 1 is substituted from asparagine (N) to tyrosine (Y); and
4) a protein identified by an amino acid sequence having 60% or more homology to the amino acid sequence set forth in SEQ ID NO: 2, the protein having immunogenicity for production of an anti-coronavirus antibody.

The S1B protein 1 (S1 B: 330-583) of SARS-COV-2 to be used in the present invention is a protein containing the amino acid sequence (SEQ ID NO: 2) from position 332 to position 526 of the amino acid sequence set forth in SEQ ID NO: 1 described above.

The S1B protein 2 (S1 B: 332-526) of SARS-COV-2 to be used in the present invention is a protein containing the amino acid sequence (SEQ ID NO: 2) from position 332 to position 526 of the amino acid sequence set forth in SEQ ID NO: 1 described above.

### (Bifidobacterium)

Herein, the *"Bifidobacterium"* refers to a microorganism belonging to the genus *Bifidobacterium.* Examples of the *Bifidobacterium* include *Bifidobacterium adolescentis, Bifidobacterium angulatum* (*B. angulatum*), *Bifidobacterium animalis* subsp. *animalis* (*B. animalis* subsp. *animalis*), *Bifidobacterium animalis* subsp. *lactis* (*B. animalis* subsp. *lactis*), *Bifidobacterium asteroides* (*B. asteroides*), *Bifidobacterium bifidum* (*B. bifidum*), *Bifidobacterium boum* (*B. boum*), *Bifidobacterium breve* (*B. breve*), *Bifidobacterium catenulatum* (*B. catenulatum*), *Bifidobacterium choerinum* (*B. choerinum*), *Bifidobacterium coryneforme* (*B. coryneforme*), *Bifidobacterium cuniculi* (*B. cuniculi*), *Bifidobacterium denticolens* (*B. denticolens*), *Bifidobacterium dentium* (*B. dentium*), *Bifidobacterium gallicum* (*B. gallicum*), *Bifidobacterium gallinarum* (*B. gallinarum), Bifidobacterium globosum* (*B. globosum*), *Bifidobacterium indicum* (*B. indicum*), *Bifidobacterium infantis* (*B. infantis*), *Bifidobacterium inopinatum* (*B. inopinatum*)*, Bifidobacterium lactis* (*B. lactis*), *Bifidobacterium longum* (*B. longum*), *Bifidobacterium magnum* (*B. magnum*), *Bifidobacterium merycicum* (*B. merycicum*), *Bifidobacterium minimum* (*B. minimum*)*, Bifidobacterium parvulorum* (*B. parvulorum*)*, Bifidobacterium pseudocatenulatum* (*B. pseudocatenulatum*)*, Bifidobacterium pseudolongum* subsp. *globosum* (*B. pseudolongum* subsp. *globosum*), *Bifidobacterium pseudolongum* subsp. *pseudolongum* (*B. pseudolongum* subsp. *pseudolongum*)*, Bifidobacterium pullorum* (*B. pullorum*)*, Bifidobacterium ruminale* (*B. ruminale*), *Bifidobacterium ruminantium* (*B. ruminantium*), *Bifidobacterium saecu*/*are* (*B. saeculare*)*, Bifidobacterium scardovii* (*B. scardovii*)*, Bifidobacterium subtile* (*B. subtile*), *Bifidobacterium suis* (*B. suis*), *Bifidobacterium thermacidophilum* (*B. thermacidophilum*)*,* and *Bifidobacterium thermophilum* (*B. thermophilum*)*.*

Of those, *Bifidobacterium adolescentis, Bifidobacterium animalis* subsp. *animalis* (*B. animalis* subsp. *animalis*), *Bifidobacterium animalis* subsp. *lactis* (*B. animalis* subsp. *lactis*), *Bifidobacterium bifidum* (*B. bifidum*), *Bifidobacterium breve* (*B. breve*), *Bifidobacterium lactis* (*B. lactis*), *Bifidobacterium longum* (*B. longum*), and *Bifidobacterium pseudolongum* subsp. *pseudolongum* (*B. pseudolongum* subsp. *pseudolongum*) are preferably used.

In addition, resistant strains or variants thereof may be used. Each of those bacterial strains is commercially available or easily available from the depository institution or the like. Examples thereof include B. *longum* 105-A, *B. longum* JCM1217 (ATCC15707), and *B. bifidum* ATCC11863.

### (Membrane Protein derived from Bacterium)

The transformed *Bifidobacterium* of the present invention designed to display the part or the whole of the constituent protein of the coronavirus as a coronavirus antigen on the surface of the *Bifidobacterium* preferably contains DNA encoding an important membrane protein derived from a bacterium that actively transports a specific substance on a cell membrane. The membrane protein derived from a bacterium is not particularly limited, but is suitably a membrane protein belonging to the ATP binding cassette protein (ABC protein) family. Specifically, as a GNB/LNB substrate-binding membrane protein (GLBP: galacto-n-biose-lacto-n-biose l-binding protein), there are given ABC proteins of the *Bifidobacterium* that transport lacto-N-biose (i.e., N-acetyl-3-O-β-D-galactopyranosyl-D-glucosamine) and galacto-N-biose (i.e., N-acetyl-3-O-(β-D-galactopyranosyl)-D-galactosamine). The GNB/LNB substrate-binding membrane protein is hereinafter sometimes referred to simply as "GLBP". ABC proteins are important membrane proteins that actively transport specific substances on the cell membranes of all organisms through use of adenosine triphosphate (ATP) as energy. Many kinds of ABC proteins are present on the cell membranes. Accordingly, the GLBP, which is a kind of ABC protein, is ubiquitously expressed through utilization of an appropriate promoter in a *Bifidobacterium* having a cellular function for expressing the GLBP on the surface thereof. Herein, the structure of the GLBP is not limited to a naturally occurring GLBP, and the GLBP may have one or more of substitutions, insertions, or deletions in its constituent amino acids as long as the GLBP has an ability to be expressed on the cell surface of the *Bifidobacterium.*

### (Fusion Protein to be displayed on Surface of Bifidobacterium)

The part or the whole of the constituent protein (e.g., the part or the whole of the S protein) of the coronavirus to be expressed and displayed on the surface of the *Bifidobacterium* of the present invention is suitably expressed as a fusion protein with the membrane protein derived from a bacterium (e.g., the GLBP). In the fusion protein, the membrane protein derived from a bacterium and the part or the whole of the constituent protein of the coronavirus are linked in the stated order from the N-terminus. As required, the fusion protein may contain a protein having an adjuvant function between the membrane protein derived from a bacterium and the part or the whole of the constituent protein of the coronavirus.

### (Preparation of Transformed Bifidobacterium)

A procedure for preparing the transformed *Bifidobacterium* expressing and displaying the part or the whole of the constituent protein of the coronavirus on the surface of the *Bifidobacterium* is described in order of operations.

### 1. Acquisition of DNA encoding each Protein

DNA encoding the membrane protein derived from a bacterium (e.g., the GLBP) may be obtained on the basis of respective known gene information or amino acid sequence information. For example, the DNA may be acquired by amplifying, through a polymerase chain reaction (PCR), genomic DNA or cDNA prepared from any bacterium (e.g., *Bifidobacterium)* serving as a template with use of a primer pair produced on the basis of genomic information on a structural gene for the membrane protein (e.g., the GLBP) of the bacterium. In general, a plurality of kinds of genetic codes exist for one amino acid, and hence a gene having a base sequence different from a known base sequence or from a base sequence based on a known amino acid sequence may be adopted. Specifically, DNA encoding the GLBP of B. *longum* may be obtained on the basis of, for example, gene information on the GLBP of B. *longum* identified in Acta Crystallographica Section F., 2007, Volume F63, p. 751. The DNA encoding the GLBP of *B*. *longum* may be obtained by amplifying, through PCR, chromosomal DNA or cDNA of *B*. *longum* serving as a template with use of a primer pair produced on the basis of gene information.

DNA encoding the part or the whole of the constituent protein of the coronavirus may be produced and obtained by a method known per se or any method to be developed in the future on the basis of the amino acid sequence information identified for the part or the whole of the constituent protein of the coronavirus described above. DNAs encoding proteins other than the part or the whole of the constituent S protein of the coronavirus described above may be similarly produced and obtained by a method known per se or any method to be developed in the future.

The full genome sequence of SARS-COV-2 is set forth in, for example, GenBank ACCESSION: MT263387.1 (SARS-CoV-2/human/USA/WA-UW304/2020). DNA encoding the S protein of SARS-COV-2 to be used for generating the transformed *Bifidobacterium* in the present invention may be identified by any one of the following items 1) to 3):
1) DNA encoding a protein obtained on the basis of amino acid sequence information identified by SEQ ID NO: 1;
2) DNA that hybridizes under stringent conditions with DNA formed of a base sequence identified by the item 1); and
3) DNA formed of a base sequence having 60% or more homology to the base sequence identified by one of the item 1) or 2).

The DNA encoding the part of the S protein of SARS-COV-2 is DNA encoding the above-mentioned S1 protein (S1: 1-681), S1AB protein (S1AB: 1-583), S1B protein 1 (S1B: 330-583), orS1B protein 2 (S1B: 332-526), and in particular, may be selected from the above-mentioned DNAs each encoding the S protein of SARS-COV-2.

The base sequence of the DNA encoding the S1 protein (S1: 1-681) out of the DNAs encoding the parts of the S protein of SARS-COV-2 is specifically shown below on the basis of the amino acid sequence set forth in GenBank ACCESSION: BCB97891.1.

· Base sequence of DNA encoding S1 protein (S1: 1-681) (SEQ ID NO: 3)

ATGTTTGTCTTCCTCGTGCTGCTCCCCCTCGTGTCGTCGCAATGTGTGAATCTCACGACCAGGAC

CCAGCTCCCGCCCGCTTACACGAATTCGTTTACCCGCGGTGTTTACTATCCGGATAAGGTGTTCCG

TAGCTCGGTCCTGCATAGCACGCAAGACCTGTTTCTCCCGTTTTTCTCCAACGTGACGTGGTTCC

ATGCGATTCACGTGTCGGGGACGAACGGCACGAAGAGGTTCGACAACCCGGTCCTGCCGTTCAA

TGATGGCGTTTATTTCGCGTCGACCGAGAAGAGCAATATTATCCGTGGTTGGATTTTTGGGACGAC

CCTGGACTCCAAAACGCAGTCGCTCCTCATTGTTAATAATGCTACCAACGTTGTGATCAAAGTTTGT

GAATTCCAGTTCTGCAACGATCCCTTTCTGGGGGTCTATTACCATAAGAATAACAAATCGTGGATGG

AATCGGAGTTTCGCGTGTATAGCTCGGCTAATAATTGTACGTTCGAATATGTCAGCCAACCCTTTCT

GATGGATCTCGAAGGTAAACAAGGTAACTTCAAAAATCTCCGTGAATTCGTTTTTAAGAACATTGAT

GGTTACTTTAAGATTTATTCGAAGCATACCCCCATTAATCTCGTGCGGGACCTCCCGCAAGGATTCA

GCGCCCTCGAGCCCCTGGTTGATCTCCCGATCGGAATTAACATCACCCGTTTTCAAACGCTGCTG

GCTCTCCATCGCTCCTACCTGACCCCCGGTGACTCGTCCTCGGGATGGACGGCGGGCGCTGCG

GCGTATTACGTGGGGTACCTGCAGCCGAGGACGTTCCTGCTGAAGTACAATGAGAATGGTACCAT

TACGGATGCTGTCGACTGTGCTCTGGACCCCCTCTCGGAAACGAAGTGCACCCTGAAGTCCTTTA

CCGTCGAAAAGGGAATCTACCAAACGAGCAATTTCAGGGTGCAACCGACCGAATCGATCGTGAGG

TTTCCCAACATCACCAACCTCTGCCCCTTCGGCGAAGTGTTCAACGCCACGCGCTTTGCCTCCGT

GTATGCTTGGAATCGTAAAAGGATCTCCAATTGCGTCGCTGACTATTCCGTTCTGTATAATTCCGCC

TCCTTTTCGACGTTCAAGTGCTATGGAGTTAGCCCCACGAAGCTCAATGACCTGTGCTTCACCAAC
GTTTATGCGGATAGCTTTGTCATCCGGGGTGACGAAGTTAGGCAAATTGCGCCCGGCCAAACCGG
TAAAATCGCTGATTACAACTATAAACTGCCCGACGATTTCACCGGTTGCGTTATCGCCTGGAATTCC
AACAATCTCGATTCGAAGGTCGGAGGTAACTATAACTATCTGTATCGTCTGTTCCGTAAATCCAATC
TGAAACCCTTTGAACGGGACATTTCCACCGAAATTTATCAAGCCGGATCCACCCCGTGTAACGGG
GTTGAAGGTTTCAACTGCTACTTCCCGCTGCAATCGTACGGATTCCAGCCCACCAATGGAGTGGG
ATATCAACCGTACCGTGTCGTGGTGCTGAGCTTCGAACTCCTGCACGCGCCCGCCACGGTGTGT
GGACCCAAGAAAAGCACGAATCTCGTGAAGAATAAATGCGTTAATTTTAACTTCAATGGGCTGACC
GGAACGGGGGTCCTCACGGAGTCCAATAAGAAATTTCTGCCCTTCCAACAGTTTGGTCGGGATAT
CGCGGATACGACCGATGCGGTCCGTGATCCGCAAACGCTGGAGATTCTCGATATCACCCCGTGTA
GCTTTGGAGGGGTCTCGGTTATCACGCCCGGGACCAACACCTCGAATCAAGTTGCTGTTCTCTAC
CAAGACGTCAATTGTACGGAGGTCCCGGTGGCTATTCATGCCGACCAGCTGACGCCCACCTGGA
GGGTCTACTCCACCGGGTCGAATGTGTTCCAGACGCGTGCCGGTTGCCTGATTGGCGCCGAGCA
CGTTAATAACAGCTATGAATGTGATATCCCGATTGGAGCTGGAATCTGCGCGTCCTACCAAACGCA
AACGAACTCCCCC

The DNA encoding each protein described above may be DNA capable of hybridizing under stringent conditions with the DNA acquired as described above. The "DNA capable of hybridizing under stringent conditions" means DNA obtained by a colony hybridization method, a plaque hybridization method, a Southern blot hybridization method, or the like through use of the above-mentioned DNA as a probe. A specific example thereof is DNA that can be identified by: performing hybridization at about 65°C in the presence of sodium chloride at from about 0.7 M to about 1.0 M with a filter having immobilized thereon DNA derived from a colony or a plaque; and then washing the filter with an SSC solution having a concentration of from about 0.1× to about 2× (the composition of an SSC solution having a concentration of 1× is formed of 150 mM sodium chloride and 15 mM sodium citrate) under the condition of about 65°C. A specific example of the DNA capable of hybridizing is DNA having at least about 80% or more homology to the base sequence of the above-mentioned DNA encoding each protein obtained on the basis of known base sequence information or amino acid sequence information, preferably DNA having about 90% or more homology thereto, more preferably DNA having about 95% or more homology thereto. DNA encoding each protein obtained on the basis of amino acid sequence information may have a different codon as long as an amino acid is encoded.

### 2. Preparation of Vector for Bifidobacterium Transformation

The preparation of recombinant DNA having the DNAs encoding the respective proteins prepared in the section 1. is described. Herein, for the recombinant DNA, an expression vector or a chromosomal integration vector (e.g., a homologous recombination vector) may be used. A plasmid to be used for the preparation of such vector is not particularly limited as long as the plasmid can be expressed in the *Bifidobacterium,* and may be a plasmid known per se or any plasmid to be developed in the future. For example, pHY, pTB6, pBL67, pBL78, pNAL8H, pNAL8M, pNAC1, pBC1, pMB1, or pGBL8b may be used as a plasmid derived from the *Bifidobacterium.* A composite plasmid of any of those plasmids and a plasmid of *E*. *coli* may be used. For example, pBLES100, pKKT427, or pRM2 may be used. From the viewpoints of the stability of expression and the ease of preparation of DNA for the preparation of a transformant, of the above-mentioned plasmids, a composite plasmid synthesized from a plasmid of *B*. *longum* and a plasmid of *E*. *coli* is suitable.

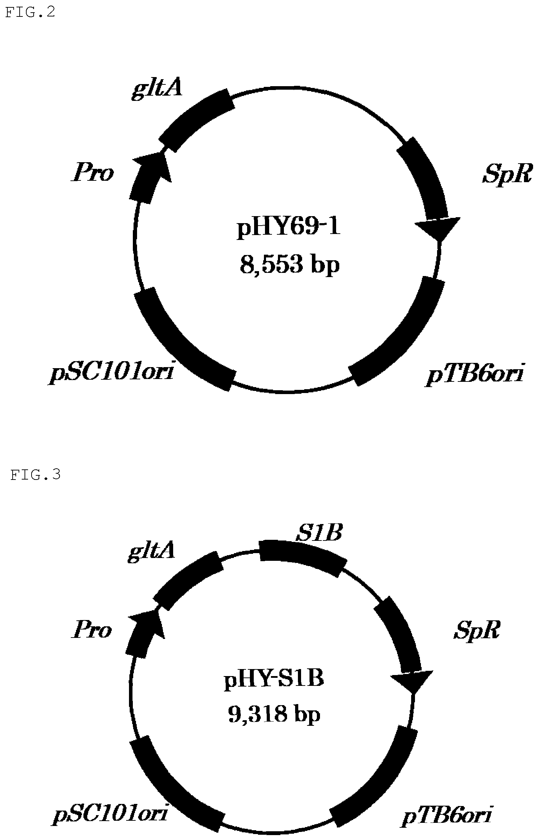

Specifically, the plasmid may be extracted using PureYield^{™} Plasmid Miniprep System (manufactured by Promega) from a *Bifidobacterium* (pHY) harboring a *Bifidobacterium-E. coli* shuttle vector (pHY69-1: FIG. 2) having the origin of replication, i.e., ori region of *E*. *coli* (pSC101ori), the origin of replication, i.e., ori region of the *Bifidobacterium* (pTB6ori), a spectinomycin resistance gene (SpR), a GLBP gene (gltA), and its promoter region (pro). The full-length DNA (vector DNA) of pHY69-1 may be amplified by performing a PCR reaction using the extracted plasmid as a template.

From the viewpoint of selecting a transformant, it is suitable that the expression vector have a selectable marker, such as antibiotic resistance or an amino acid requirement, on the basis of a method known per se. The expression vector preferably has added thereto a regulatory sequence in order to express the fusion protein of the membrane protein derived from a bacterium (e.g., the GLBP) and the part or the whole of the constituent protein of the coronavirus, or so as to be advantageous for the expression. Examples of the regulatory sequence include a promoter sequence, a leader sequence, a propeptide sequence, an enhancer sequence, a signal sequence, and a terminator sequence. The origin of each of those regulatory sequences is not particularly limited as long as the regulatory sequence is expressed in the *Bifidobacterium.* The promoter sequence is not particularly limited as long as the promoter sequence is expressed in the *Bifidobacterium.* From the viewpoint of expression efficiency, the promoter sequence of the histone-like protein (HU) of *B*. *longum,* the LDH promoter thereof, or the like is preferably used. In addition, from the viewpoint of enhancing expression efficiency, the expression vector preferably has a terminator sequence. As the terminator sequence, the terminator sequence of the HU gene is preferably used. In addition, DNA encoding a linker having an appropriate length may be arranged between the DNA encoding the bacterial membrane protein and the DNA encoding the part or the whole of the constituent protein of the coronavirus.

A cloning vector is prepared by introducing, as required, a regulatory sequence, such as a promoter sequence or a terminator sequence, and a selectable marker gene into the above-mentioned plasmid, as described above. Examples of the selectable marker include: antibiotic resistance markers, such as spectinomycin (SPr), ampicillin (Ampr), tetracycline (TETr), kanamycin (KMr), streptomycin (STr), and neomycin (NEOr); fluorescent markers, such as a green fluorescent protein (GFP) and a red fluorescent protein (REP); and enzymes such as LacZ. The cloning vector preferably has, for example, a linker having a multiple cloning site downstream of its promoter. Through use of such linker, the DNA encoding the fusion protein is integrated downstream of the promoter and so as to allow in-frame expression of the fusion protein. As the plasmid for the cloning vector, pBLES100, pBLEM100, or the like may be typically used.

In-frame integration of the acquired HU promoter sequence, DNA encoding the membrane protein derived from a bacterium, and DNA encoding the part or the whole of the constituent protein of the coronavirus into the plasmid pBLES100 can produce a vector for expressing the fusion protein on the surface of the *Bifidobacterium.* The expression vector produced by such method is used for the transformation of the *Bifidobacterium.*

### 3. Preparation of Transformed Bifidobacterium expressing Protein

The recombinant DNA, for example, the expression vector is transferred into the *Bifidobacterium* serving as a host. As a transformation method, a method known per se or any method to be developed in the future may be applied. Specific examples thereof include an electroporation method, a calcium phosphate method, a lipofection method, a calcium ion method, a protoplast method, a microinjection method, and a particle gun method. In the present invention, the electroporation method is preferably used. When the electroporation method is adopted, the method may be performed under the conditions of from 0.5 kV/cm to 20 kV/cm and from 0.5 µsec to 10 msec. It is desired that the electroporation method be more preferably performed under the conditions of from 2 kV/cm to 10 kV/cm and from 50 µsec to 5 msec.

The transformant may be selected using the selectable marker of the protein expression vector (preferably a fusion protein expression vector) as an indicator. Examples of a medium for culturing the transformant include media each suitable for each host microorganism, such as a glucose blood liver (BL) agar medium, a de Man, Rogosa and Sharpe (MRS) agar medium, a Gifu Anaerobic (GAM) agar medium, an improved GAM (TGAM) agar medium, a Briggs agar medium, a yeast extract glucose peptone (YGP) agar medium, and AnaeroPack^{™} Kenki (manufactured by Mitsubishi Gas Chemical Inc.).

The transformant is preferably cultured under anaerobic culture conditions under which the *Bifidobacterium* can be cultured. When the culture is performed under anaerobic conditions, the growth of aerobic bacteria can be prevented. The anaerobic conditions are culture in a hermetic vessel capable of keeping anaerobicity that allows the growth of the *Bifidobacterium,* and examples thereof include conditions that can be established in an anaerobic chamber, an anaerobic box, or the like. A culture temperature only needs to be a temperature at which the *Bifidobacterium* can be cultured, and is generally from 4°C to 45°C, preferably from 15°C to 40°C, more preferably from 24°C to 37°C.

The expression of the protein in the obtained transformed *Bifidobacterium* may be determined by a method known per se that is applied in gene recombination technology or any method to be developed in the future. The expression may be determined by, for example, a western blotting method. The western blotting method may also be performed by a method known per se. In particular, for example, that the part or the whole of the S protein of the coronavirus is displayed on the surface of the *Bifidobacterium* can be easily determined by subjecting the transformed *Bifidobacterium* to an immunoantibody method involving using, for example, an antibody against the part or the whole of the S protein of the coronavirus and an FITC-labeled anti-IgG antibody. In the case of coexpressing the membrane protein derived from a bacterium (e.g., the GLBP), a protein having an adjuvant function (e.g., CD40 ligand: CD40L), and the part or the whole of the S protein of the coronavirus, the protein having an adjuvant function and the part or the whole of the S protein of the coronavirus are displayed on the surface of the *Bifidobacterium,* and hence the antibody to be used for the determination may be an antibody against any of the proteins.

The transformed *Bifidobacterium* that has been determined as displaying the part or the whole of the constituent protein of the coronavirus on the surface thereof may be cultured, recovered, and used as it is for the production of a formulation, by methods to be generally used by a person skilled in the art. The obtained *Bifidobacterium* may be inactivated by heat sterilization treatment, radiation irradiation, or the like before use. The transformed *Bifidobacterium* may be subjected to post-treatment by a known method. For example, partial purification may be performed by centrifugation or the like. In addition, as desired, after the partial purification has been performed, the transformed *Bifidobacterium* may be dissolved or suspended in a solvent that has been conventionally used in the art, such as saline, phosphate-buffered saline (PBS), or lactated Ringer's solution. In addition, as desired, the transformed *Bifidobacterium* may be freeze-dried or spray-dried to be turned into a powdery product or a granular product.

### (Formulation containing Transformed Bifidobacterium)

The present invention also encompasses a coronavirus infectious disease vaccine formulation containing the transformed *Bifidobacterium* as an active ingredient of a coronavirus infectious disease vaccine. When the part or the whole of the constituent protein of the coronavirus displayed on the surface is administered for the purpose of treating or preventing a disease, the transformed *Bifidobacterium* serving as the active ingredient may be administered in the form of any formulation. An administration route is not particularly limited, and for example, oral administration or parenteral administration may be performed, but oral administration is suitable because of the purpose of the present invention.

Examples of the formulation suitable for oral administration include a tablet, a granule, a fine granule, a powder, a syrup, a solution, a capsule, and a suspension. Examples of the formulation suitable for parenteral administration include an injection, an infusion, an inhalant, an aerosol, a suppository, a transdermal preparation, and a transmucosal preparation.

In the production of a liquid formulation for oral administration, there may be used an additive for formulation, for example: water; a sugar, such as sucrose, sorbitol, or fructose; a glycol, such as polyethylene glycol or propylene glycol; an oil, such as sesame oil, olive oil, or soybean oil; or a preservative such as a p-hydroxybenzoic acid ester. In addition, in the production of a solid formulation, such as a capsule, a tablet, a powder, or a granule, there may be used, for example: an excipient, such as lactose, glucose, sucrose, or mannitol; a disintegrant, such as starch or sodium alginate; a lubricant, such as magnesium stearate or talc; a binder, such as polyvinyl alcohol, hydroxypropyl cellulose, or gelatin; a surfactant such as a fatty acid ester; or a plasticizer such as glycerin.

### (Oral Vaccine)

The transformed *Bifidobacterium* displaying the part or the whole of the constituent protein of the coronavirus of the present invention is an active ingredient of a coronavirus infectious disease vaccine, and can be suitably utilized as an oral vaccine. For example, the part or the whole of the constituent protein of the coronavirus is recognized as an antigen on the wall of the intestinal tract, resulting in production of an antibody. Accordingly, the transformed *Bifidobacterium* can serve as an effective oral vaccine. For example, each of acid-resistant capsule formulations described below (a seamless capsule formulation, a soft capsule formulation, and a hard capsule formulation), when orally administered, passes through the stomach having a pH of from 1 to 3, without dissolving therein, to reach the intestines, and dissolves in the intestines. The transformed *Bifidobacterium* released from the formulation through the dissolution of the capsule maintains most protein structures even in the intestinal environment and displays the part or the whole of the constituent protein of the coronavirus on the surface thereof.

When the transformed *Bifidobacterium* is orally administered, the part or the whole of the constituent protein of the coronavirus, which is expressed on the surface of the *Bifidobacterium,* is taken up by gut-associated lymphoid tissue (GALT), and is processed with an appropriate epitope by an antigen-presenting cell (APC), such as a dendritic cell or a B cell, in the GALT. Further, it is conceived that a peptide subjected to the processing in the APC is displayed on the surface of the APC together with MHC class II or MHC class I, and induces a CD8-positive T cell (CTL) having a T cell receptor specific to the peptide. It is conceived that the APC activates CD4-positive T cells (Th1 cells), and the actions of various cytokines, such as IFN-γ and IL-2, released from the CD4-positive T cells, allow the growth of CD8-positive T cells specific to the S protein. Meanwhile, for example, CD4-positive T cells (Th2 cells) react to an antigen epitope displayed together with MHC class II by B cells that have taken up an S protein antigen to secrete cytokines, such as IL-4, IL-5, and IL-6, causing the antigen-presenting B cells to differentiate into plasma cells and grow, with the result that an anti-S protein antibody is produced. The part or the whole of the constituent protein of the coronavirus of the present invention activates both the CD8-positive T cells and the CD4-positive T cells, and hence is conceived to efficiently exhibit a cellular and humoral immune action on the coronavirus. The present invention also encompasses a method of administering a coronavirus infectious disease vaccine formulation based on oral administration.

The coronavirus infectious disease vaccine formulation containing the transformed *Bifidobacterium* of the present invention as an active ingredient may include an adjuvant, or may be administered together with an adjuvant. The adjuvant has an action of boosting the effect of the vaccine. The adjuvant to be used for the coronavirus infectious disease vaccine formulation of the present invention is preferably an adjuvant capable of boosting the induction of mucosal immunity, and examples thereof include, but not limited to, aluminum hydroxide and inorganic salts thereof, hydrocarbons, such as squalene and oil, bacterial toxins, such as cholera toxin, *E. coli* heat-labile enterotoxin B subunit (LTB), and lipid A derived from a bacterium (MPLA), polysaccharides, such as chitosan and inulin, nucleic acids, such as dsRNA, CpG ODN, and a STING ligand, and cations, such as DOTAP and DDA, as well as cyclodextrins, and combinations thereof. Further, the transformed *Bifidobacterium* of the present invention that is caused to coexpress a protein having an adjuvant function may be used as the active ingredient. Examples of the protein having an adjuvant function include CD40 ligands (CD40L, TRAP, and CD154). CD40L is a ligand protein belonging to the TNF superfamily, and is expressed in one of a 33 kDa transmembrane form or an 18 kDa soluble form (sCD154).

The present invention relates to a method of preventing or treating a coronavirus infectious disease including a step of administering the coronavirus infectious disease vaccine formulation of the present invention to a subject. The present invention also encompasses a method of preventing coronaviral infection and/or a method of preventing an increase in severity after coronaviral infection. Further, the present invention also encompasses a method of preventing and/or treating a sequela after coronaviral infection. The dose of the active ingredient varies depending on, for example, the body weight and age of the subject, symptoms, and an administration method, but could be appropriately selected by a person skilled in the art. The prevention and/or treatment of coronaviral infection and/or an increase in severity after coronaviral infection, a sequela after coronaviral infection, etc. in the present invention is based on the fact that the oral vaccine of the present invention has an effect of boosting humoral and/or cellular immunity against the coronavirus. The present invention further also encompasses a method of boosting immunity including administering the coronavirus infectious disease vaccine formulation of the present invention. More specifically, the present invention also encompasses, for example, a method of boosting humoral immunity and/or a method of boosting cellular immunity.

### (Production of Acid-resistant Capsule Formulation containing Transformed Bifidobacterium)

The coronavirus infectious disease vaccine formulation of the present invention preferably has the form of a capsule formulation. Herein, a capsule containing a content is referred to as "capsule formulation". The capsule formulation in the present invention includes a capsule coating and the transformed *Bifidobacterium* expressing the part or the whole of the constituent protein of the coronavirus on the surface thereof, and the capsule coating is acid-resistant. The capsule formulation including the acid-resistant capsule coating and the transformed *Bifidobacterium* expressing the part or the whole of the constituent protein of the coronavirus on the surface thereof may have any configuration and shape as long as the capsule formulation has the acid-resistant capsule coating and contains as a capsule content, the transformed *Bifidobacterium* expressing the part or the whole of the constituent protein of the coronavirus on the surface thereof. It is not excluded that the capsule formulation includes an additional constituent element. Accordingly, the transformed *Bifidobacterium* expressing the part or the whole of the constituent protein of the coronavirus on the surface thereof is included or encapsulated in the acid-resistant capsule coating (i.e., contained in the internal region of a capsule formed by the acid-resistant coating). The capsule formulation applicable to the transformed *Bifidobacterium* of the present invention may be produced using a method known per se or any method to be developed in the future.

In order for the transformed *Bifidobacterium* expressing the part or the whole of the constituent protein of the coronavirus on the surface thereof to function as an oral vaccine for a coronavirus infectious disease, it is required that: the transformed *Bifidobacterium* pass through the stomach to reach the intestines; and the protein that is the part or the whole of the constituent protein of the coronavirus and cell wall proteins of the *Bifidobacterium* be maintained even in the intestines. Incidentally, the pH of the stomach is from 1 to 3, and most proteins of the orally ingested *Bifidobacterium* are denatured owing to the markedly low pH. Accordingly, in order that the transformed *Bifidobacterium* to be used in the present invention may reach the human intestines while maintaining various protein structures, and display the part or the whole of the constituent protein of the coronavirus, it is preferred that the transformed *Bifidobacterium* be prevented from being affected by gastric acid to the extent possible.

To that end, in the present invention, it is suitable to adopt a capsule formulation in which the transformed *Bifidobacterium* is included or encapsulated in an acid-resistant capsule coating, i.e., the transformed *Bifidobacterium* is contained inside a capsule formed of the acid-resistant coating. The configuration, shape, and the like of the capsule formulation are not particularly limited as long as the coating has resistance to gastric acid. That is, the capsule formulation is desirably configured such that gastric acid is prevented from entering the inside of the capsule to be brought into contact with the transformed *Bifidobacterium.* The capsule coating may be a coating that does not dissolve at a pH of 4 or less, preferably at a pH of from 1 to 3. A capsulation method is also not particularly limited.

### Examples

The present invention is specifically described below by way of Examples. However, the present invention is not limited to Examples below. In Examples described below, a *Bifidobacterium* expressing a fusion protein of a partial protein of the S protein of SARS-COV-2 and a GNB/LNB substrate-binding membrane protein (GLBP) on the surface thereof was generated. The S protein of SARS-CoV-2 has the amino acid sequence identified by 1,273 amino acids (SEQ ID NO: 1) disclosed in GenBank ACCESSION: BCB97891.1. In Examples, for the partial protein of the S protein of SARS-COV-2 in the fusion protein, the expression of each of an S1 protein (S1: 1-681), an S1AB protein (S1AB: 1-583), an S1B protein 1 (S1B: 330-583), an S1B protein 2 (S1B: 332-526), and an N501Y-type S1B protein 3 (S1B: 332-526) was tried.

### (Example 1) Generation of Bifidobacterium expressing S Protein of Coronavirus on Surface thereof 1

In this Example, for the partial protein of the S protein of SARS-COV-2 in the fusion protein, a transformed *Bifidobacterium* for each fusion protein expression expressing each of the S1 protein (S1: 1-681), the S1AB protein (S1AB: 1-583), and the S1B protein 1 (S1B: 330-583) was generated (see FIG. 1).

### A. Isolation of Vector DNA (Bifidobacterium-E. coli Shuttle Vector (pHY69-1))

A plasmid was extracted using PureYield^{™} Plasmid Miniprep System (manufactured by Promega) from a *Bifidobacterium* (pHY) harboring a *Bifidobacterium-E. coli* shuttle vector (pHY69-1: FIG. 2) having the origin of replication, i.e., ori region of *E*. *coli* (pSC101ori), the origin of replication, i.e., ori region of the *Bifidobacterium* (pTB6ori), a spectinomycin resistance gene (SpR), a GLBP gene (gltA), and its promoter region (pro). A PCR reaction was performed using the extracted plasmid as a template to amplify the full-length DNA (vector DNA) of pHY69-1. The amplified PCR products were subjected to agarose electrophoresis, and an 8,553 bp PCR product was excised, and isolated and purified using Wizard SV Gel and PCR Clean-Up System (manufactured by Promega).

The following were used as primers for the PCR reaction.
pSC101 End F: 5'-TATGCACAGATGAAAACGGTG-3' (SEQ ID NO: 4)
GLBP End R: 5'-AGTACTCTCGGAAACAGACAGG-3' (SEQ ID NO: 5)

### B. Isolation of Insert DNA

An S1 gene encoding the S1 protein (S1: 1-681) of SARS-CoV-2 (GenBank: BCB97891.1) was totally synthesized (Eurofins Genomics) (SEQ ID NO: 3). Codons frequently used in *Bifidobacterium* were used in the synthesis. For the S1 protein (S1: 1-681), the S1AB protein (S1AB: 1-583), and the S1B protein 1 (S1B: 330-583), a PCR reaction was performed using the S1 gene as a template and using primers for each protein expression and KOD-Plus Neo (manufactured by TOYOBO) to amplify a DNA fragment (insert DNA) encoding each protein. A stop codon was added to the C-terminus side (TAA: SEQ ID NO: 6). The amplified PCR products were subjected to agarose gel electrophoresis, and 2,076 bp, 1,782 bp, and 795 bp PCR products were excised for S1 (1-681), S1AB (1-583), and S1B (330-583), respectively, and were isolated and purified using Wizard SV Gel and PCR Clean-Up System (manufactured by Promega).

The following were used as primers for the PCR reaction having the bases of a fusion portion added upstream and downstream of DNA encoding each protein for a surface expression *Bifidobacterium.*

### · Primers for GLBP-S1 Fusion Gene

S1-Infusion F: 5'-GTTTCCGAGAGTACTATGTTTGTCTTCCTCGTGC-3' (SEQ ID NO: 7)
S1-Infusion R: 5'-TTTCATCTGTGCATATTAGGGGGAGTTCGTTTGC-3' (SEQ ID NO: 8)

### · Primers for GLBP-S1AB Fusion Gene

S1AB-lnfusion F: 5'-GTTTCCGAGAGTACTATGTTTGTCTTCCTCGTGC-3' (SEQ ID NO: 9)
StAB-infusion R: 5'-TTTCATCTGTGCATATTACTCCAGCGTTTGCGG-3' (SEQ ID NO: 10)

### · Primers for GLBP-S1B-330-583 Fusion Gene

S1B-330-583 Infusion F: 5'-GTTTCCGAGAGTACTCCCAACATCACCAACCTC-3' (SEQ ID NO: 11)
S1B-330-583 Infusion R: 5'-TTTCATCTGTGCATATTACTCCAGCGTTTGCGG-3' (SEQ ID NO: 12)

### C. Production of Plasmid carrying Gene encoding Partial Protein of S Protein

The vector DNA of the section A. and each insert DNA generated in the section B. (gene encoding a partial protein of the S protein) were subjected to in-fusion using In-Fusion^{™} HD Cloning Kit (manufactured by Takara Bio), and the obtained plasmid was transferred into E. *coli* HB101 Competent Cells (manufactured by Takara Bio) by a heat shock method. The resultant was applied to an LB agar medium containing 50 µg/ml of spectinomycin, and was cultured at 37°C overnight to provide transformed E. *coli* harboring a plasmid having the origin of replication, i.e., ori region of E. *coli* (pSC101ori), the origin of replication, i.e., ori region of the *Bifidobacterium* (pTB6ori), a spectinomycin resistance gene (SpR), the gene encoding the partial protein of the S1 protein, a GLBP gene (gltA), and its promoter region (pro) (see FIG. 3 for S1B-330-583).

The plasmid was extracted and purified from the transformed E. *coli* using PureYield^{™} Plasmid Miniprep System (manufactured by Promega), and the presence of each gene sequence was recognized. The obtained recombinant plasmids for S1 protein expression, for S1AB protein expression, and for S1B protein 1 expression were named pHY-S1, pHY-S1AB, and pHY-S1B-330-583, respectively.

### D. Generation of Bifidobacterium expressing Fusion Protein of GLBP and each Protein on Surface thereof by Transformation of Bifidobacterium with each Recombinant Plasmid

*Bifidobacterium longum* 105-A (JCM 31944) was inoculated on 5 ml of a Gifu anaerobic medium (GAM) (manufactured by Nissui Pharmaceutical Co., Ltd.), and was cultured at 37°C overnight using AnaeroPack^{™} Kenki (manufactured by Mitsubishi Gas Chemical Company, Inc.). The cultured *B. longum* 105A was inoculated on 30 ml of a GAM medium, and was cultured at 37°C using AnaeroPack^{™} Kenki. An absorbance at a wavelength of 600 nm was measured during the culture, and the culture was stopped at the time point when the absorbance reached about 0.5. After the completion of the culture, centrifugation was performed with a high-speed centrifuge at 9,000 rpm and 4°C for 10 minutes to collect the bacteria. The collected bacterial cells were washed two or three times by adding 10 ml of a TE buffer (0.05 M Scrose, 1 mM Triammonium Citrate, pH 6.0) to suspend the bacterial cells and centrifuging the suspension with a high-speed centrifuge. 200 µl of the suspension containing the washed bacterial cells was taken in a separate tube, and 10 µl of each of solutions containing the respective recombinant plasmids obtained in the section C. was added. The contents were mixed, and the whole was left to stand still on ice. Then, the mixed liquid was placed in a 0.2 cm Gene Pulser^{™}/Micropulser^{™} electroporation cuvette (manufactured by Bio-Rad) and subjected to electroporation using a Gene Pulser Xcell^{™} electroporation system (manufactured by Bio-Rad) under the conditions of 2,000 V, 25 µF, and 200 Ω. Immediately after the electroporation, 1.0 ml of a GAM medium that had been adjusted to 37°C in advance was added, and the cells were cultured at 37°C for 3 hours using AnaeroPack^{™} Kenki. Then, the resultant was applied to a GAM agar medium containing 50 µg/ml of spectinomycin (manufactured by Nissui Pharmaceutical Co., Ltd.), and was cultured at 37°C using AnaeroPack^{™} Kenki to provide a transformed *Bifidobacterium.* The resultant transformed *Bifidobacterium* was inoculated on a GAM medium containing 50 µg/ml of spectinomycin, and was cultured at 37°C using AnaeroPack^{™} Kenki. After the completion of the culture, the culture broth was dispensed into a 1.5 ml tube to generate a 20% glycerol stock, which was frozen and stored at -80°C.

Each obtained transformed *Bifidobacterium* was used as master cells of a *Bifidobacterium* expressing the fusion protein of the GLBP and each protein on the surface thereof. The transformed *Bifidobacterium* for each fusion protein expression was named B-COV-S1for the S1 protein, B-COV-S1AB for the S1AB protein, and B-COV-S1B-330-583 for the S1B protein 1. Further, the respective fusion proteins predicted to be expressed were named GLBP-S1, GLBP-S1AB, and GLBP-S1B-330-583, respectively.

### (Example 2) Generation of Bifidobacterium expressing S protein of Coronavirus on Surface thereof 2

In this Example, for the partial protein of the S protein of SARS-COV-2 in the fusion protein, a transformed *Bifidobacterium* for fusion protein expression expressing the S1B protein 2 (S1B:332-526) was generated by the same technique as that of Example 1.

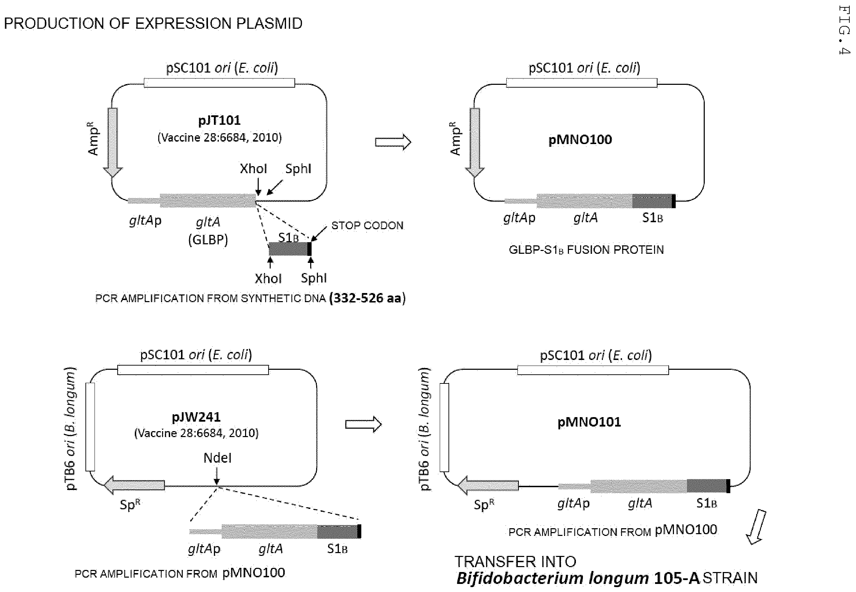

A. A plasmid was extracted using PureYield^{™} Plasmid Miniprep System (manufactured by Promega) from *E*. *coli* harboring an E. *coli* vector (pJT101: FIG. 4) having the origin of replication, i.e., ori region of *E*. *coli* (pSC101ori), an ampicillin resistance gene (AmpR), a GLBP gene (gltA), and its promoter region (pro).

### B. Isolation of Insert DNA

The S1B protein 2 (S1B:332-526) of SARS-CoV-2 (GenBank: BCB97891.1) was amplified by PCR using the S1 region plasmid generated in Example 1 as a template.

### · Primers for S1B-332-526 Gene

S1B-332-526 F: 5'-gcactcgagatcaccaacctctgcccct-3' (SEQ ID NO: 13)
S1B-332-526 R: 5'-ccagcatgcttcatccacacaccgtggcgg-3' (SEQ ID NO: 14)

### C. Production of Plasmid carrying Gene encoding Partial Protein of S Protein

The vector DNA of the section A. and each insert DNA generated in the section B. (gene encoding a partial protein of the S protein) were fused to the C-terminus side of the GLBP gene on pJT101. Specifically, a gene fragment of about 600 bases amplified with Primer S1B-332-526 F and Primer S1B-332-526 R was ligated to a pJT101 fragment treated with Xhol and Sphl. A DNA ligation kit Mighty Mix (manufactured by Takara Bio) was used for the ligation reaction. *E. coli* DH5alpha was transformed using the reaction liquid, and the resultant plasmid was subjected to sequence analysis to determine the sequence of each GLBP-S1B-332-526 fusion gene, and was named pMNO100.

### D. Generation of Bifidobacterium expressing Fusion Protein of GLBP and each Protein on Surface thereof by Transformation of Bifidobacterium with each Recombinant Plasmid

The GLBP-S1B-332-526 fusion gene containing a promoter region was amplified by PCR and introduced into the *Bifidobacterium-E. coli* shuttle vector pJW241. Specifically, a gene fragment of about 2,400 bases amplified with Primer S1B-332-526 Infusion F and Primer S1B-332-526 Infusion R was inserted into the Ndel region of pJW241. In-fusion method (manufactured by Takara Bio) was used for the insertion. *E. coli* DH5alpha was transformed using the reaction liquid, and the resultant plasmid was named pMNO101 after the sequence of the GLBP-S1B-332-526 fusion gene therein had been determined (see FIG. 4). The resultant pMNO101 was transferred into the B. *longum* 105A strain by an electroporation method to generate a recombinant *Bifidobacterium.* The recombinant was selected by spectinomycin resistance (30 µg/ml).

### · Primers for GLBP-S1B-332-526 Fusion Gene

S1B-332-526 Infusion F: 5'-ggaaaactgtccatacatatgggcgatggcgagg-3' (SEQ ID NO: 15)
S1B-332-526 Infusion R: 5'-tttcatctgtgcatagcatgcttcatccacacaccg-3' (SEQ ID NO: 16)

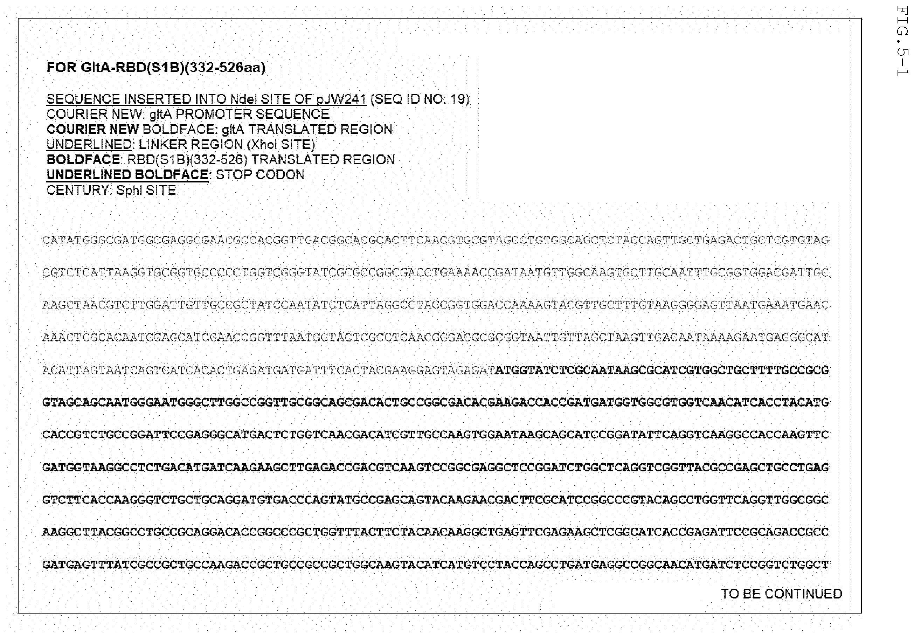

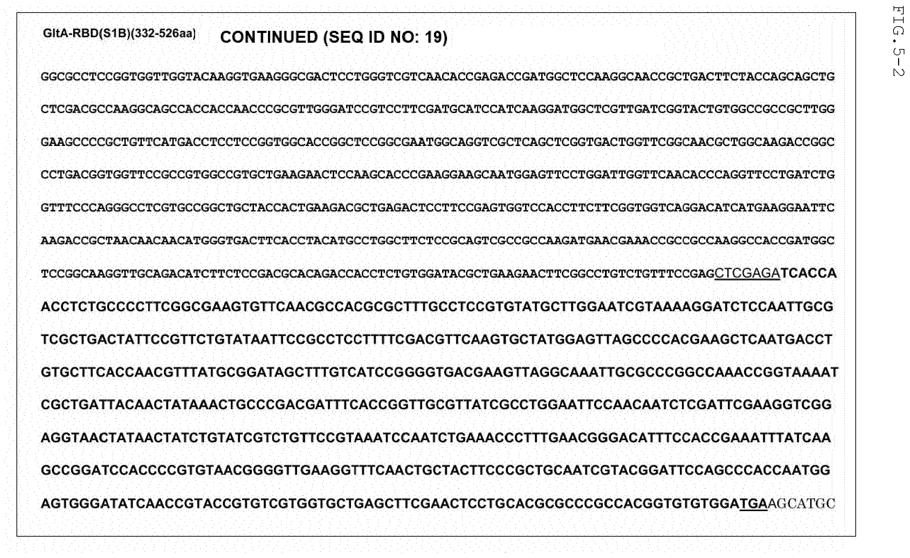

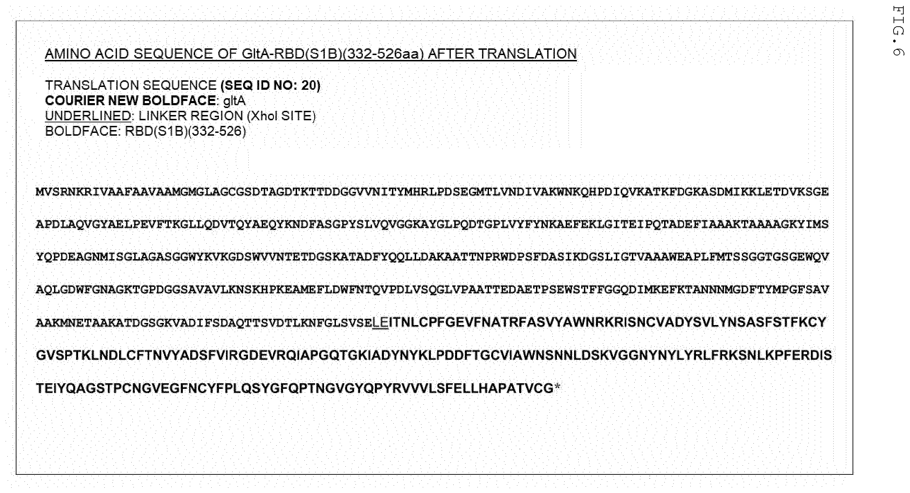

The GLBP-S1B-332-526 fusion gene containing a promoter region introduced into the above-mentioned *Bifidobacterium-E. coli* shuttle vector pJW241 is identified by a base sequence set forth in SEQ ID NO: 19 (FIG. 5-1 and FIG. 5-2), and its amino acid sequence after translation is identified by an amino acid sequence set forth in SEQ ID NO: 20 (FIG. 6).

Each obtained transformed *Bifidobacterium* was used as master cells of a *Bifidobacterium* expressing a fusion protein of the GLBP and each protein on the surface thereof. The obtained transformed *Bifidobacterium* was treated by the same technique as that of Example 1, and was frozen and stored at -80°C. The transformed *Bifidobacterium* for fusion protein expression was named B-COV-S1B-332-526. Further, each fusion protein predicted to be expressed was named GLBP-S1B-332-526.

### (Example 3) Generation of Bifidobacterium expressing S protein of Coronavirus on Surface thereof 3

In this Example, for the partial protein of the S protein of SARS-COV-2 in the fusion protein, a transformed *Bifidobacterium* for fusion protein expression expressing the N501Y-type S1B protein 3 (S1B:332-526) was generated.

The N501Y-type S1B protein 3 (S1B: 332-526) was produced by a mutagenic polymerase reaction using pMNO101 produced in the section D. of Example 2 as a template. An elongation reaction was performed using S1B-332-526 N501Y F and S1B-332-526 N501Y shown below as a primer pair, and the resultant reaction liquid was treated with a restriction enzyme Dpnl to remove the template plasmid, followed by transfer into *E*. *coli* DH5α. The plasmid was extracted from the recombinant, and after the gene sequence of the N501Y-type S1B protein 3 (S1B: 332-526 N501Y) had been determined, was transferred into B. *longum* 105-A by electroporation.

### · S1B: Primers for 332-526 N501Y Gene

S1B-332-526 N501Y F: 5'-ttccagcccacctacggagtgggatat-3' (SEQ ID NO: 17)
S1B-332-526 N501Y R: 5'-atatcccactccgtaggtgggctggaa-3' (SEQ ID NO: 18)

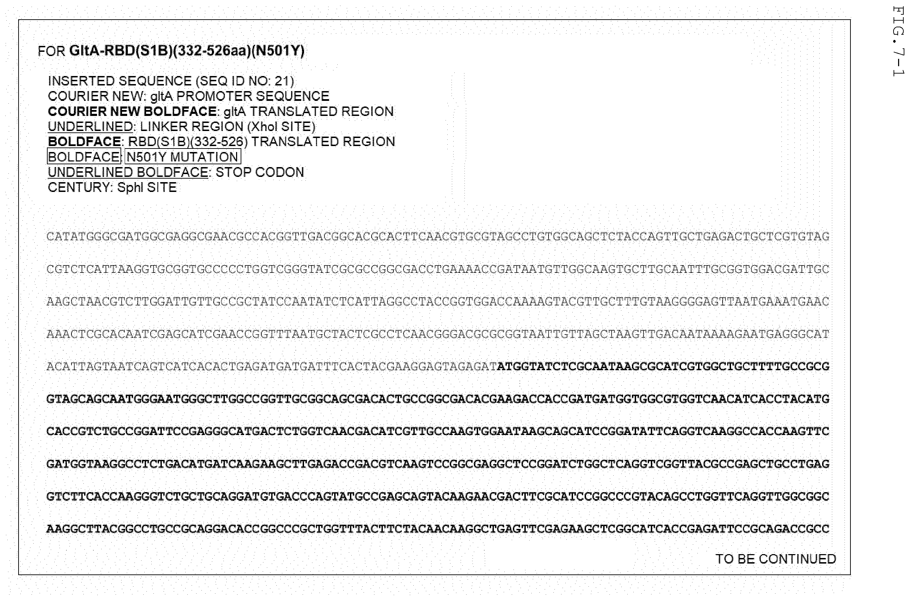

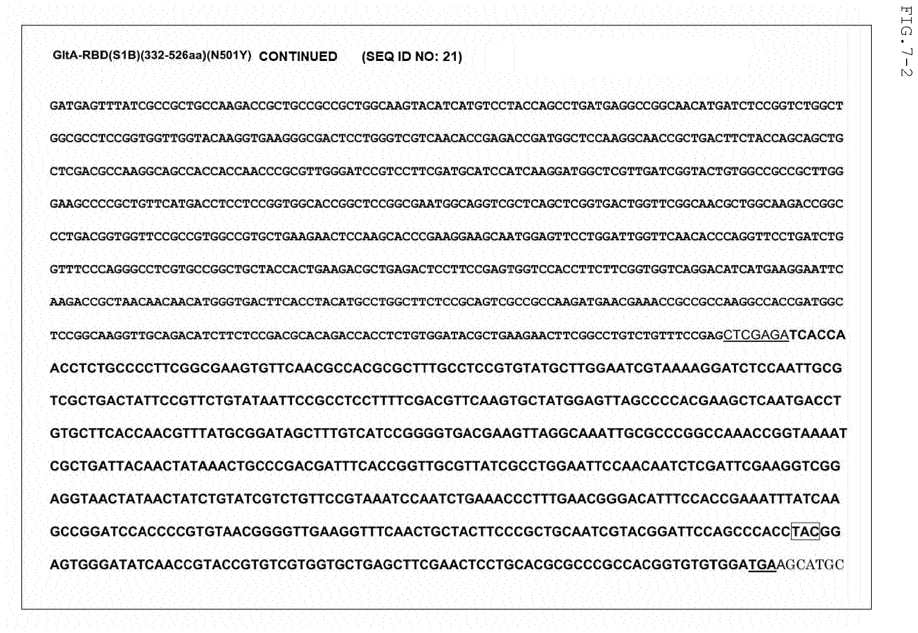

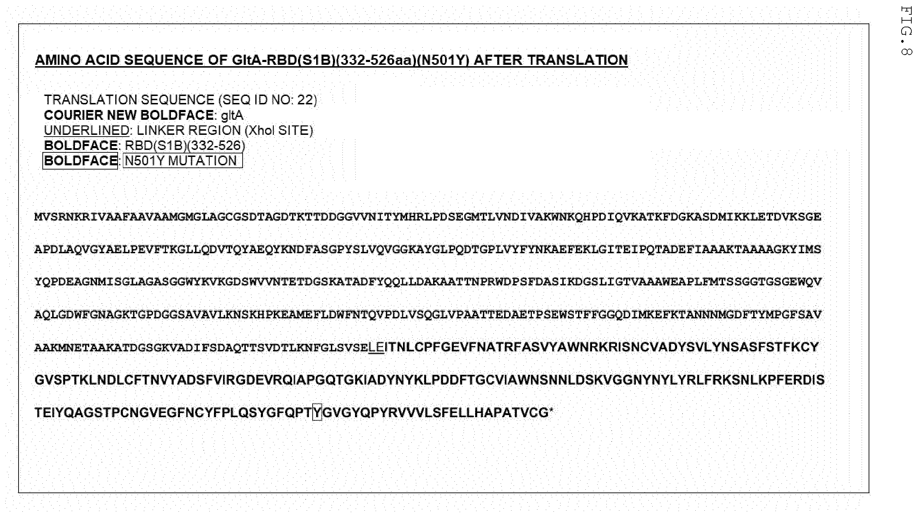

The GLBP-S1B-332-526 N501Y fusion gene containing a promoter region introduced into the above-mentioned *Bifidobacterium-E. coli* shuttle vector pJW241 is identified by a base sequence set forth in SEQ ID NO: 21 (FIG. 7-1 and FIG. 7-2), and its amino acid sequence after translation is identified by an amino acid sequence set forth in SEQ ID NO: 22 (FIG. 8).

Each obtained transformed *Bifidobacterium* was used as master cells of a *Bifidobacterium* expressing a fusion protein of the GLBP and each protein on the surface thereof. The obtained transformed *Bifidobacterium* was treated by the same technique as that of each of Examples 1 and 2, and was frozen and stored at -80°C. The transformed *Bifidobacterium* for fusion protein expression was named B-COV-S1B-332-526 N501Y. Further, each fusion protein predicted to be expressed was named GLBP-S1B-332-526 N501Y.

### (Example 4) Determination of Surface Expression of each Fusion Protein in Transformed Bifidobacterium 1

In this Example, for each transformed *Bifidobacterium* generated in Example 1 and Example 2, the surface expression of the fusion protein of the GLBP and each partial protein of the S protein of SARS-COV-2 was determined by a western blotting method. The expression of the partial protein of the S protein was determined using antibodies against the receptor binding domain (RBD) of the S protein and the GLBP.

Each transformed *Bifidobacterium* generated in Example 1 and Example 2 described above was subjected to centrifugation treatment with a high-speed centrifuge to collect bacterial cells of each kind. The collected bacterial cells were washed three times by adding PBS to suspend the bacterial cells and using a high-speed centrifuge. To the washed bacterial cells, 1 ml of an 8 M urea buffer and 1 µl of 1 M dithiothreitol were added to prepare a bacterial cell suspension, which was left to stand on ice for 30 minutes. The bacterial cell suspension was subjected to ultrasonic disruption treatment on ice using Vibra-Cell^{™} VCX 130 (manufactured by Sonics & Materials) under the conditions of an output of 100%, a pulse of 10 seconds, and an interval of 20 seconds. This was repeated 10 times to provide a bacterial cell disruption liquid. The bacterial cell disruption liquid was centrifuged with a high-speed centrifuge at 9,000 rpm and 4°C for 10 minutes to provide a protein supernatant. A buffer for a sample (for SDS-PAGE, 6× concentrated, containing a reducing agent) (manufactured by Nacalai Tesque, Inc.) was added to the supernatant, and the mixture was left to stand at 95°C for 5 minutes to provide a sample for electrophoresis. Then, e-PAGEL^{™} (10-20%) (ATTO Corporation) was set in an electrophoresis apparatus (ATTO Corporation), and the sample was applied and subjected to electrophoresis along with a molecular weight marker at a current of 20 mA for 1.5 hours. The gel after the electrophoresis was placed on a Poly Vinylidene Di-Fluoride (PVDF) membrane (manufactured by Cytiva), and blotting treatment was performed by applying a current of 20 mA to a blotting apparatus (manufactured by Bio-Rad).

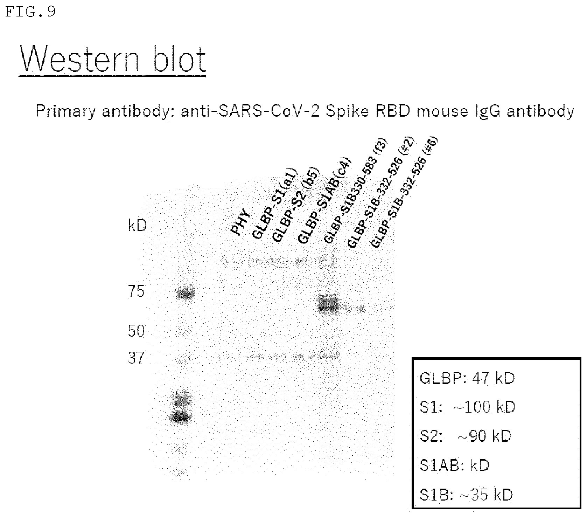

After the blotting, the PVDF membrane was washed twice with PBS-T (manufactured by Takara Bio), and subjected to blocking treatment with Blocking One (manufactured by Nacalai Tesque, Inc.) by being shaken at room temperature for 30 minutes. The PVDF membrane subjected to the blocking treatment was washed twice with PBS-T, and shaken overnight in 3% bovine serum albumin (BSA)-PBS having added thereto 0.4% (v/v) of a primary antibody (Anti-SARS-CoV-2 Spike RBD Mab (Clone 1034522) (manufactured by R&D SYSTEMS)). The PVDF membrane was further washed twice with PBS-T, and then shaken for 1 hour in 3% BSA-PBS having added thereto 0.02% (v/v) of a secondary antibody (Anti-IgG (H+L chain) (Mouse) pAb-HRP (manufactured by MBL)). The PVDF membrane was washed three times with PBS-T, and surface expression of the fusion protein of the GLBP and each protein was determined through color development with Chemi-Lumi One L (manufactured by Nacalai Tesque, Inc.). As a result, detection with the anti-RBD antibody was found for the transformed *Bifidobacterium* identified as each of B-COV-S1B-330-583 and B-COV-S1B-332-526 (FIG. 9).

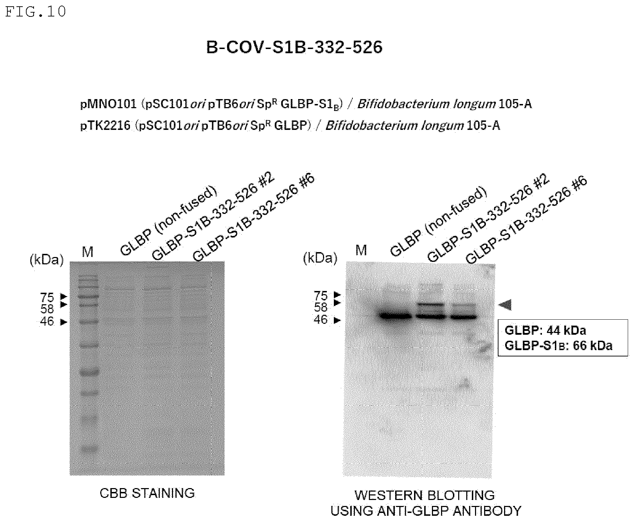

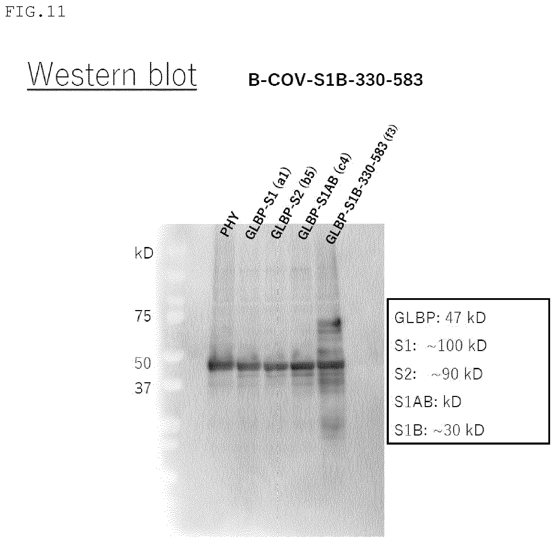

Further, a PVDF membrane treated in the same manner as above was shaken overnight in 3% BSA-PBS having added thereto 0.05% (v/v) of a primary antibody (Rat anti-GLBP monoclonal IgG antibody). The PVDF membrane was further washed twice with PBS-T, and then shaken for 1 hour in 3% BSA-PBS having added thereto 0.02% (v/v) of a secondary antibody (Goat Anti-Rat IgG H&L (HRP) (manufactured by Abcam). The PVDF membrane was washed three times with PBS-T, and surface expression of the fusion protein of the GLBP and each protein was determined through color development with Chemi-Lumi One L (manufactured by Nacalai Tesque, Inc.). As a result, detection of the GLBP was found for the transformed *Bifidobacterium* identified as each of B-COV-S1B-330-583 and B-COV-S1B-332-526, and the displayed protein was recognized to be the fusion protein of each partial protein of the S protein of SARS-COV-2 and the GLBP (FIG. 10 and FIG. 11).

### (Example 5) Determination of Surface Expression of each Fusion Protein in Transformed Bifidobacterium 2

In this Example, surface expression of the fusion protein of the GLBP and each partial protein of the S protein of SARS-COV-2 in the transformed *Bifidobacterium* was determined by a fluorescent cell staining method.

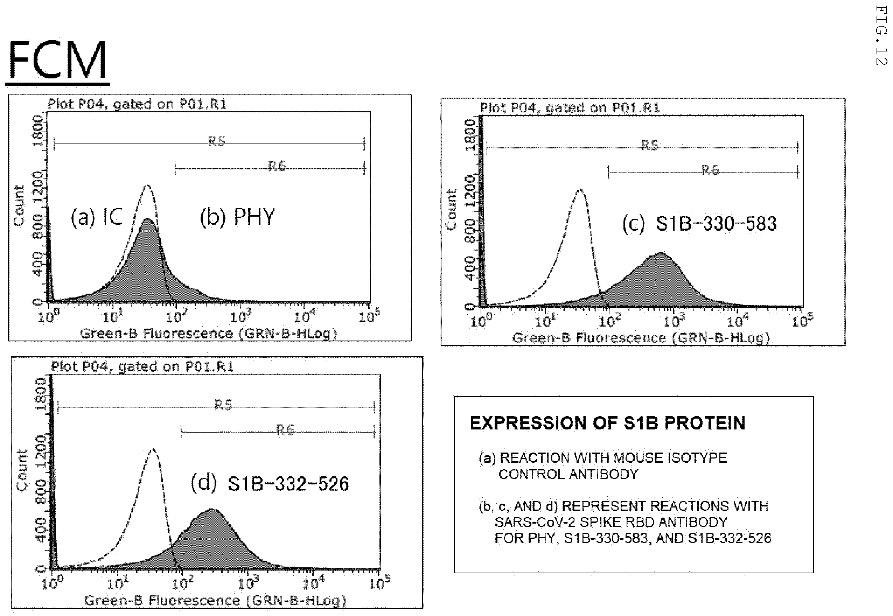

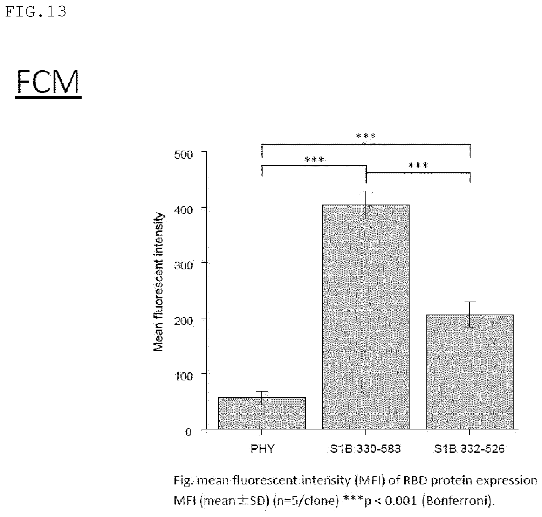

Of the frozen and stored transformed *Bifidobacteriums* generated in Examples 1 and 2 described above, B-COV-S1B-330-583 and B-COV-S1B-332-526, and PHY were each inoculated on a GAM agar medium containing 50 µg/ml of spectinomycin (manufactured by Nissui Pharmaceutical Co., Ltd.), and cultured at 37°C using AnaeroPack^{™} Kenki. A colony on the agar medium was inoculated on a GAM medium containing 50 µg/ml of spectinomycin, and cultured at 37°C using AnaeroPack^{™} Kenki. After the culture, 100 µl of the bacterial liquid, which had been adjusted to OD=1.0 through measurement of its absorbance at 600 nm, was transferred to a 1.5 ml tube. The bacterial liquid was centrifuged with a high-speed centrifuge at 10,000 rpm and 4°C for 5 minutes. The supernatant was removed with an aspirator, the bacterial cells were suspended by adding PBS thereto, and the suspension was centrifuged with a high-speed centrifuge at 10,000 rpm and 4°C for 5 minutes. The supernatant was removed with an aspirator, and the cells were suspended by adding 300 µl of Blocking One (manufactured by Nacalai Tesque, Inc.), followed by incubation at room temperature for 15 minutes. After the incubation, 1 ml of PBS was added, and each mixture was subjected to centrifugation treatment with a high-speed centrifuge at 10,000 rpm and 4°C for 5 minutes to collect bacterial cells of each kind. The collected bacterial cells were washed twice by adding PBS to suspend the bacterial cells again and using a high-speed centrifuge. The bacterial cells from which the supernatant had been removed were suspended by adding thereto 100 µl of Can Get Signal^{™} immunostain Solution A (manufactured by TOYOBO) having added thereto 2% (v/v) of a primary antibody (anti-RBD antibody, Anti-SARS-CoV-2 Spike RBD Mab: Clone 1034522 (manufactured by R&D SYSTEMS), followed by incubation at room temperature for 1 hour. After the incubation, 1 ml of PBS was added, and each mixture was subjected to centrifugation treatment with a high-speed centrifuge at 10,000 rpm and 4°C for 5 minutes to collect bacterial cells of each kind. The collected bacterial cells were washed twice by adding PBS to suspend the bacterial cells again and using a high-speed centrifuge. The bacterial cells from which the supernatant had been removed were suspended by adding thereto 100 µl of Can Get Signal^{™} immunostain Solution A (manufactured by TOYOBO) having added thereto 0.05% (v/v) of a secondary antibody (Alexa Fluor^{™} 488 goat anti-Mouse IgG (H+L) (manufactured by Invitrogen)) or an isotype control (FITC Rat IgG2a, κ Isotype Ctrl Antibody (manufactured by BioLegend)), followed by incubation at room temperature under a light-shielding condition for 1 hour. After the incubation, 1 ml of PBS was added, and each mixture was subjected to centrifugation treatment with a high-speed centrifuge at 10,000 rpm and 4°C for 5 minutes to collect bacterial cells of each kind. The collected bacterial cells were washed twice by adding PBS to suspend the bacterial cells again and using a high-speed centrifuge. The supernatant was removed, and the cells were suspended by adding 500 µl of PBS. The suspension was used as a sample for measurement and analyzed with a flow cytometer using analysis software included therewith (see FIG. 12 and FIG. 13).

The results were as follows: PHY showed no change in anti-RBD antibody-positive cell count with respect to the isotype control, but B-COV-S1B-330-583 and B-COV-S1B-332-526 showed clear increases in anti-RBD antibody-positive cell count (FIG. 12). In addition, B-COV-S1B-330-583 and B-COV-S1B-332-526 showed significantly high values in mean fluorescent intensity with respect to the anti-RBD antibody as compared to PHY (FIG. 13). It was able to be recognized from the above-mentioned results that B-COV-S1B-330-583 and B-COV-S1B-332-526 each expressed a partial protein of the S protein of SARS-COV2 on the surface thereof.

### (Example 6) Determination of Humoral Immune Response-inducing Effect of Oral Administration of Transformed Bifidobacterium

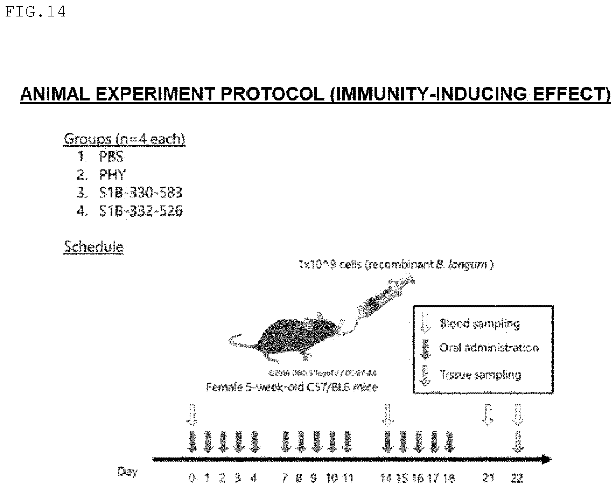

A humoral immune response-inducing effect achieved when, of the frozen and stored transformed *Bifidobacteriums* generated in Examples 1 and 2 described above, B-COV-S1B-330-583 and B-COV-S1B-332-526 were each orally administered to mice (C57/BL6, ♀, 5-week-old) was determined. A solution of each transformed *Bifidobacterium* (1×10⁹) in 100 µl of PBS was orally administered on Days 0 to 4, 7 to 11, and 14 to 18 with n=4 for each (see FIG. 14).

### (1) With regard to IgG Antibody Production

For sera from blood collected from the tail vein on Days 0, 14, and 21 from the initiation of the administration, the concentrations of an anti-S1B-RBD protein IgG antibody against the RBD portion contained in the S1B protein were measured by an enzyme-linked immuno sorbent assay (ELISA) method. The measurement was performed using RayBio^{™} COVID-19 S1 RBD protein Human IgG ELISA Kit (manufactured by RayBiotech) by a method in conformity with the manual of the kit. 100 µl of a serially diluted human anti-S1B-RBD protein IgG antibody (positive control) provided in the kit and 100 µl of each serum diluted 50-fold with 1×Sample Diluent were added to a 96-well plate coated with SARS-CoV-2 RBD protein, and the whole was shaken at room temperature for 1 hour. The solution in each of the wells was discarded, and the wells were washed four times with 1×Wash buffer. 100 µl of Biotinylated Anti-Human IgG Antibody was added to the positive control, 100 µl of 0.02% (v/v) Anti-IgG (H+L chain) (Mouse) pAb-HRP (manufactured by MBL) was added to each of the samples, and the whole was shaken at room temperature for 1 hour. After the 1 hour, the solution in each of the wells with only the positive control was discarded, the wells were washed four times with 1×Wash buffer, and 100 µl of an HRP-streptavidin solution was added, followed by further shaking at room temperature for 30 minutes. After the 30 minutes, the solution in all the wells was discarded, and the wells were washed four times with 1×Wash buffer. 100 µl of TMB One-Step Substrate Reagent was added to each well, and the whole was shaken at room temperature under a light-shielding condition for 15 minutes. 50 µl of Stop Solution was added to each well, and an absorbance at 450 nm was measured.

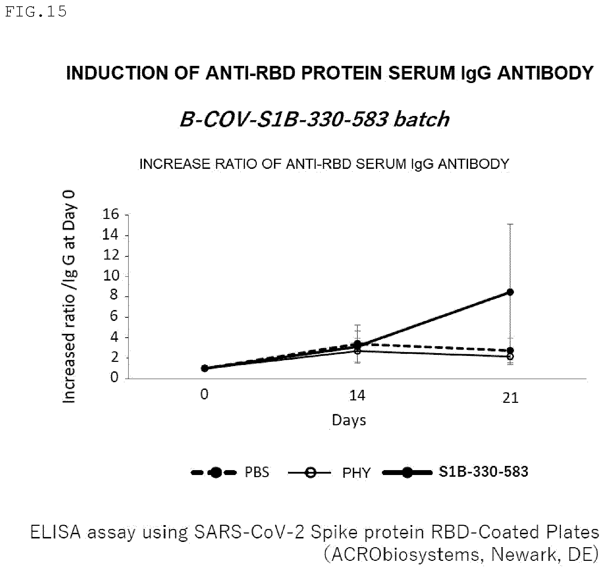

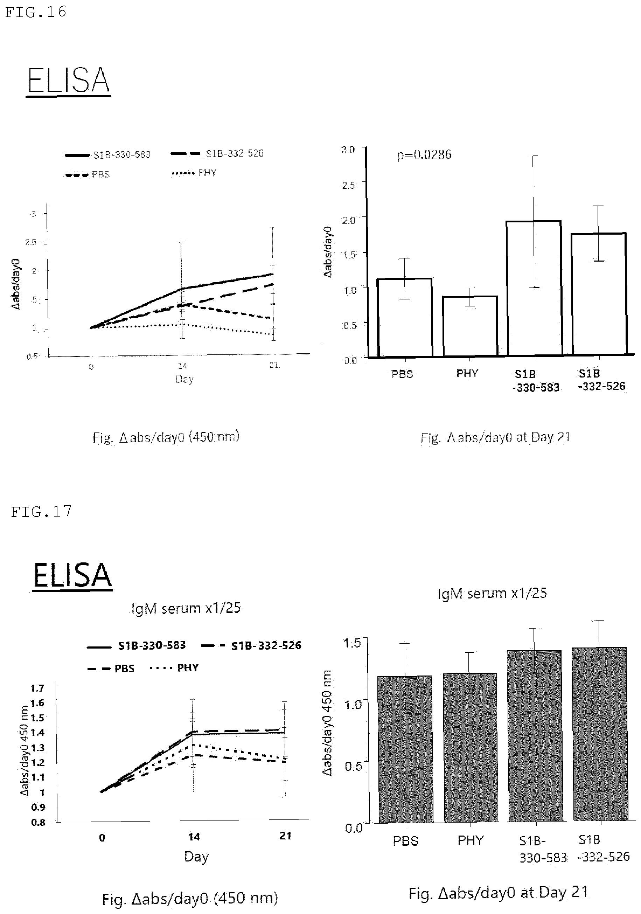

As a result, it was recognized that the concentration of the anti-S1B-RBD protein IgG antibody was increased in each of the sera of the mice to which the transformed *Bifidobacteriums* identified as B-COV-S1B-330-583 and B-COV-S1B-332-526 had been orally administered (FIG. 15 and FIG. 16). Thus, it was recognized that the transformed *Bifidobacteriums* identified as B-COV-S1B-330-583 and B-COV-S1B-332-526 each had the potential to serve as an active ingredient showing a vaccine effect on a coronavirus infectious disease, especially COVID-19.

### (2) With regard to IgM Production

A humoral immune response-inducing effect achieved when, of the frozen and stored transformed *Bifidobacteriums* generated in Example 1 described above, B-COV-S1B-330-583 and B-COV-S1B-332-526 were each orally administered to mice (C57/BL6, ♀, 5-week-old) was determined on the basis of IgM production. A solution of each transformed *Bifidobacterium* (1×10⁹) in 100 µl of PBS was orally administered on Days 0 to 4, 7 to 11, and 14 to 18 with n=4 for each (see FIG. 14).

For sera from blood collected from the tail vein on Days 0, 14, and 21 from the initiation of the administration, the concentrations of an anti-RBD IgM antibody against the RBD portion contained in the S1B protein were measured by an ELISA method. The measurement was performed using RayBio^{™} COVID-19 S1 RBD protein Human IgG ELISA Kit (manufactured by RayBiotech) by a method in conformity with the manual of the kit. 100 µl of a serially diluted human anti-RBD protein Ig G antibody (positive control) provided in the kit and 100 µl of each serum diluted 25-fold with 1×Sample Diluent were added to a 96-well plate coated with SARS-CoV-2 RBD protein, and the whole was shaken at room temperature for 1 hour. The solution in each of the wells was discarded, and the wells were washed four times with 1 ×Wash buffer. 100 µl of Biotinylated Anti-Human IgG Antibody was added to the positive control, 100 µl of 0.01% (v/v) Goat anti-Mouse IgM Antibody Biotinylated (Bethyl Laboratories) was added to each of the samples, and the whole was shaken at room temperature for 1 hour. After the 1 hour, the solution in each of the wells was discarded, and the wells were washed four times with 1×Wash buffer. 100 µl of an HRP-Streptavidin solution was added to the positive control, 100 µl of 0.01% (v/v) streptavidin (HRP) (ab7403) (manufactured by Abcam) was added to the sample, and the whole was shaken at room temperature for 1 hour. After the 1 hour, the solution in each of the wells was discarded, and the wells were washed four times with 1×Wash buffer. 100 µl of TMB One-Step Substrate Reagent was added to each well, and the whole was shaken at room temperature under a light-shielding condition for 15 minutes. 50 µl of Stop Solution was added to each well, and an absorbance at 450 nm was measured.

As a result, it was recognized that the concentration of the anti-S1B-RBD protein IgM antibody was increased in each of the sera of the mice to which the transformed *Bifidobacteriums* identified as B-COV-S1B-330-583 and B-COV-S1B-332-526 had been orally administered (FIG. 17). Thus, it was recognized that the transformed *Bifidobacteriums* identified as B-COV-S1B-330-583 and B-COV-S1B-332-526 each had the potential to serve as an active ingredient showing a vaccine effect on a coronavirus infectious disease, especially COVID-19.

### (Example 7) Determination of Cellular Immune Response-inducing Effect of Oral Administration of Transformed Bifidobacterium

A cellular immune response-inducing effect achieved when mice were subjected to oral administration on the basis of the same animal experimental protocol as that of Example 6 (FIG. 14) was determined.

On Day 22 from the initiation of the administration, the spleen was recovered from the mice, and splenocytes were prepared and cultured. The prepared splenocytes were subjected to intracellular cytokine staining (ICCS) to determine the proportion of cytokine (IFN-γ)-producing CD4-positive T cells or CD8-positive T cells in the splenocytes. The mouse splenocytes (2×10⁶ cells/well, n=5) were mixed with each SARS-CoV-2 epitope peptide shown below (MBL037 or MBL045) (manufactured by MBL) at 10 µg/ml per well, and were cultured in a 96-well plate under the conditions of 37°C and 5% CO₂ for 36 hours.
· SARS-CoV-2 epitope peptide, MBL037: GNYNYLYRL (SEQ ID NO: 23)
   Chain A, SARS-CoV-2 spike glycoprotein (447-455)
· SARS-CoV-2 epitope peptide, MBL045: YNYLYRLF (SEQ ID NO: 24)
   Chain A, SARS-CoV-2 spike glycoprotein (449-456)

GolgiStop (manufactured by BD) containing monensin as an intracellular protein transport inhibitor was added to each well with the above-mentioned cultured splenocytes, and the cells were further cultured for 12 hours. The cells were recovered, and subjected to intracellular cytokine staining using BD/Cytofix/Cytoperm Plus Fixation/Permeabilization Kit (manufactured by BD). PerCP Hamster Anti-Mouse CD3e (manufactured by BD), APC Rat Anti-Mouse CD8a (manufactured by BD), and FITC Rat Anti-Mouse CD4 (manufactured by BD) were added, followed by mixing. The cells were washed with a buffer for staining. PE Rat Anti-Mouse IFN-γ (manufactured by BD) was added to the cells, and the cells were gently suspended. After that, the whole was left to stand still in a dark place at room temperature. The cells were washed, and then resuspended in a buffer for staining. After that, the cells were analyzed with a flow cytometer using analysis software included therewith. A specific method was performed in conformity with the manual of the kit.

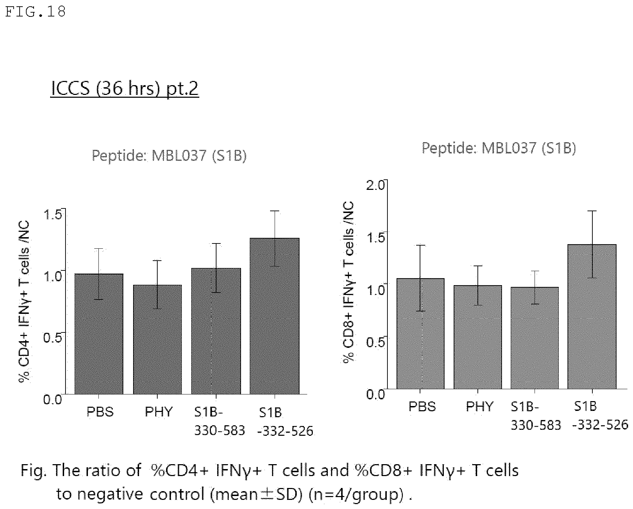

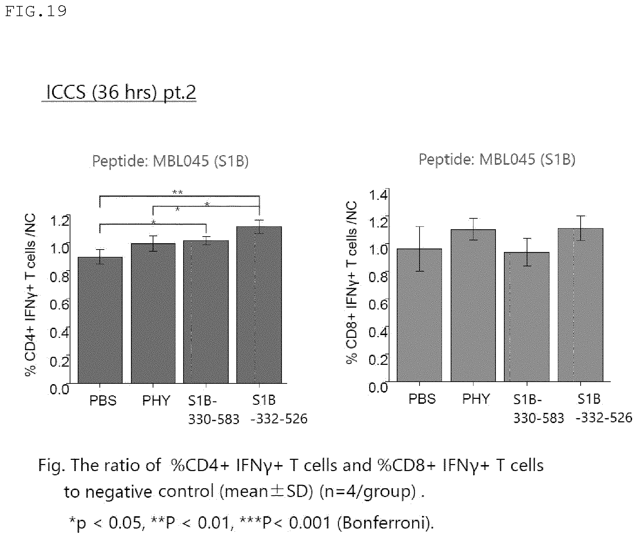

As a result, particularly in the spleen of the mice to which the transformed *Bifidobacterium* identified as B-COV-S1B-332-526 had been orally administered, there was shown a tendency that the appearance of CD4 and CD8 was boosted in both cases (FIG. 18 and FIG. 19). Thus, it was recognized that, in particular, the transformed *Bifidobacterium* identified as B-COV-S1B-332-526 had an action of enabling the boosting of S1B protein-specific cellular immunity, and had the potential to serve as an active ingredient showing a vaccine effect on a coronavirus infectious disease, especially COVID-19.

### (Example 8) Determination of Cellular Immune Response-inducing Effect of Oral Administration of Transformed Bifidobacterium 2

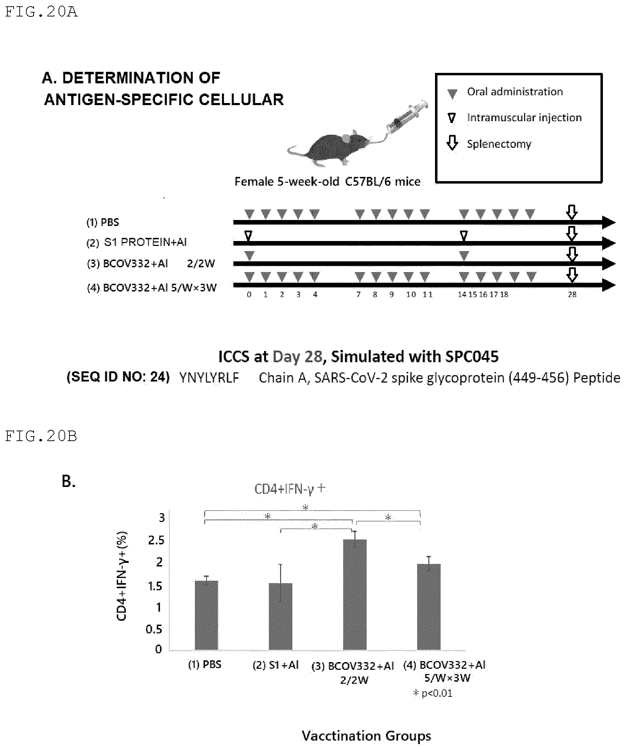

A cellular immune response-inducing effect achieved when the transformed *Bifidobacterium* B-COV-S1B-332-526 generated in Example 2 described above was orally administered to mice (C57BL/6, ♀, 5-week-old) was determined. The experiment was performed in accordance with an administration protocol illustrated in FIG. 20A with n=4 in each of the following systems (1) to (4). Specifically, oral administration was performed on Days 0 to 4, 7 to 11, and 14 to 18 in each of (1) and (4), intramuscular injection administration was performed on Days 0 and 14 in (2), and oral administration was performed on Days 0 and 14 in (3). The S1 protein of (2) was purchased from SinoBiological, Inc.
(1) PBS
(2) S1+Al: 5 µg of S1 protein+100 µl of PBS containing aluminum hydroxide adjuvant (Alum: Alhydrogel^{™} adjuvant 2%, InvivoGen)
(3) BCOV332+Al 2/2W: B-COV-S1B-332-526 (1×10⁹)+100 µl of PBS containing aluminum hydroxide adjuvant (InvivoGen)
(4) BCOV332+Al 5/W×3W: same as in (3)

On Day 28 from the initiation of the administration, the spleen was recovered from the mice, and splenocytes were prepared and cultured. The prepared splenocytes were subjected to intracellular cytokine staining (ICCS) to determine the proportion of cytokine (IFN-γ)-producing CD4-positive T cells or CD8-positive T cells in the splenocytes. The mouse splenocytes (2×10⁶ cells/well, n=5) were mixed with a SARS-CoV-2 epitope peptide shown below (MBL045) (manufactured by MBL) at 10 µg/ml per well, and were cultured in a 96-well plate under the conditions of 37°C and 5% CO₂ for 36 hours.

· SARS-CoV-2 epitope peptide, MBL045: YNYLYRLF (SEQ ID NO: 24)
Chain A, SARS-CoV-2 spike glycoprotein (449-456)

GolgiStop (manufactured by BD) containing monensin as an intracellular protein transport inhibitor was added to each well with the above-mentioned cultured splenocytes in the same manner as in Example 7, and the cells were further cultured for 12 hours. The cells were recovered, and subjected to intracellular cytokine staining using BD/Cytofix/Cytoperm Plus Fixation/Permeabilization Kit (manufactured by BD). PerCP Hamster Anti-Mouse CD3e (manufactured by BD), APC Rat Anti-Mouse CD8a (manufactured by BD), and FITC Rat Anti-Mouse CD4 (manufactured by BD) were added, followed by mixing. The cells were washed with a buffer for staining. PE Rat Anti-Mouse IFN-γ (manufactured by BD) was added to the cells, and the cells were gently suspended. After that, the whole was left to stand still in a dark place at room temperature. The cells were washed, and then resuspended in a buffer for staining. After that, the cells were analyzed with a flow cytometer using analysis software included therewith. A specific method performed was in conformity with the manual of the kit.

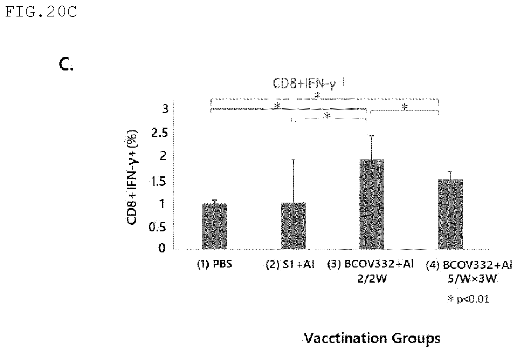

As a result, particularly in the spleen of the mice of (3) and (4) to which B-COV-S1B-332-526 had been orally administered, there was shown a tendency that the appearance of CD4 and CD8 was boosted, and (3) showed a slightly higher tendency (FIG. 20B and FIG. 20C). Thus, it was recognized that, in particular, B-COV-S1B-332-526 boosted cellular immunity in an S1B protein-specific manner by being orally administered together with the aluminum hydroxide adjuvant on Days 0 and 14, and had the potential to serve as an active ingredient showing a vaccine effect on a coronavirus infectious disease, especially COVID-19.

### (Example 9) Humoral Immune Response-inducing Effect of Oral Administration of Transformed Bifidobacterium

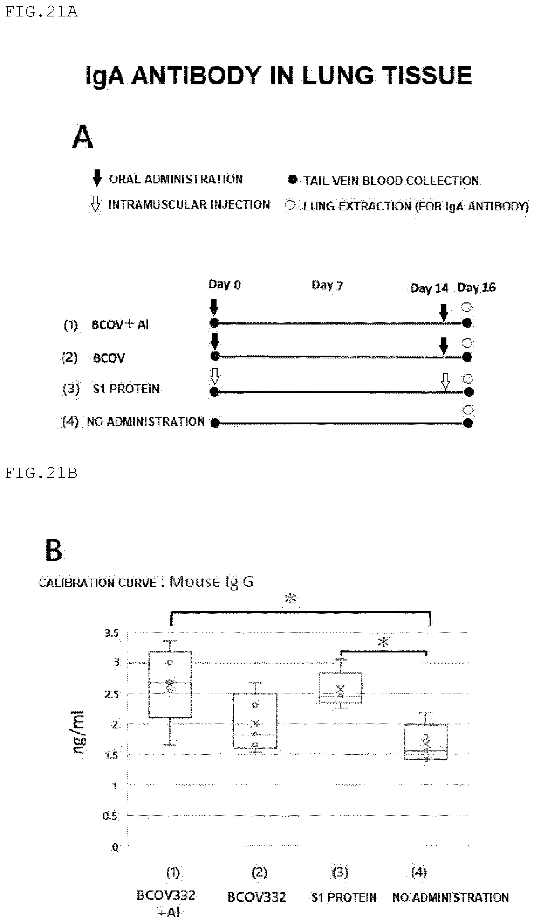

A humoral immune response-inducing effect based on IgA antibody production in lung tissue achieved when the transformed *Bifidobacterium* B-COV-S1B-332-526 generated in Example 2 described above was orally administered to mice (C57BL/6, ♀, 5-week-old) was determined. The experiment was performed in accordance with an administration protocol illustrated in FIG. 21A in each of the following systems (1) to (4). Specifically, oral administration was performed on Days 0 and 14 in each of (1) and (2), and intramuscular injection administration was performed on Days 0 and 14 in (3). (4) was a control with no administration.
(1) BCOV332+Al: B-COV-S1B-332-526 (1×10⁹+PBS containing aluminum hydroxide adjuvant (Alum: Alhydrogel^{™} adjuvant 2%, InvivoGen) (100 µl)
(2) BCOV332: B-COV-S1B-332-526 (1×10⁹)+PBS (100 µl)
(3) S1: 5 µg of S1 protein+PBS (100 µl)
(4) No administration

For each of (1) to (4), lung tissue harvested on Day 16 from the initiation of the administration was homogenized and then suspended with PBS, and the suspension was centrifuged to collect a supernatant. For IgA contained in the supernatant, the concentration of an anti-S1B-RBD protein IgA antibody against the RBD portion contained in the S1B protein was measured by an ELISA method. RayBio^{™} COVID-19 S1 RBD protein Human IgG ELISA Kit (manufactured by RayBiotech) was used. The secondary antibody for IgA measurement was changed from the one included with the kit to Biotin anti-mouse IgA (manufactured by BioLegend), but measurement was performed by a method in conformity with the manual of the kit in any other respect. 100 µl of a serially diluted human anti-S1B-RBD protein IgG antibody (positive control) and 100 µl of each lung tissue extract diluted 5-fold with 1 ×Sample Diluent were added to a 96-well plate coated with SARSCoV-2 RBD protein, and the whole was shaken at room temperature for 1 hour. The solution in each of the wells was discarded, and the wells were washed four times with 1 ×Wash buffer. 100 µl of Biotinylated Anti-Human IgG Antibody was added to the positive control, 100 µl of 0.02% (v/v) Biotin anti-mouse IgA (manufactured by BioLegend) was added to each of the samples, and the whole was shaken at room temperature for 1 hour. After the 1 hour, the solution in each of the wells was discarded, the wells were washed four times with 1 ×Wash buffer, and 100 µl of an HRP-streptavidin solution was added, followed by further shaking at room temperature for 30 minutes. After the 30 minutes, the solution in all the wells was discarded, and the wells were washed four times with 1 ×Wash buffer. 100 µl of TMB One-Step Substrate Reagent was added to each well, and the whole was shaken at room temperature under a light-shielding condition for 15 minutes. 50 µl of Stop Solution was added to each well, and an absorbance at 450 nm was measured.

As a result, a significant increase in IgA antibody in the lung tissue was able to be recognized in each of the systems (1) and (3) (FIG. 21B). Thus, it was recognized that, when the transformed *Bifidobacterium* B-COV-S1B-332-526 was orally administered on Days 0 and 14 together with the aluminum hydroxide adjuvant, acquisition of humoral immunity based on IgA production in an S1B protein-specific manner was recognized at a level comparable to that in the case of intramuscular injection administration of the S1B protein, and the transformed *Bifidobacterium* had the potential to serve as an active ingredient showing a vaccine effect on a coronavirus infectious disease, especially COVID-19.

### (Example 10) Humoral Immune Response-inducing Effects of Addition of Various Adjuvants

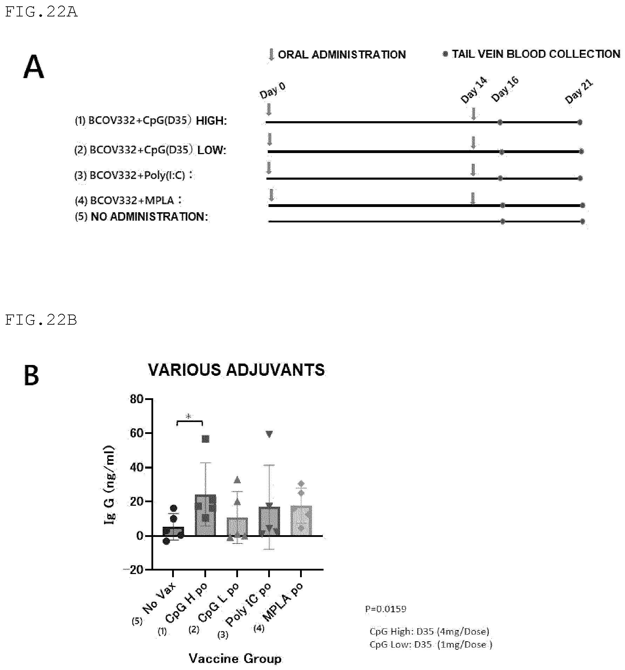

Humoral immune response-inducing effect based on IgG antibody production achieved when the transformed *Bifidobacterium* B-COV-S1B-332-526 generated in Example 2 described above was orally administered together with various adjuvants to mice (C57BL/6, ♀, 5-week-old) were determined. The experiments were performed in accordance with an administration protocol illustrated in FIG. 22A in the following systems (1) to (5), in each of which oral administration was performed on Days 0 and 14. (5) was a control with no administration.
(1) BCOV332+CpG(D35) High: B-COV-S1B-332-526 (1×10⁹)+PBS containing 4 mg of CpG(D35) (100 µl)
(2) BCOV332+CpG(D35) Low: B-COV-S1B-332-526 (1×10⁹)+PBS containing 1 mg of CpG(D35) (100 µl)
(3) BCOV332+Poly(I:C): B-COV-S1B-332-526 (1×10⁹)+PBS containing 1 mg of Poly(I:C) (100 µl)
(4) BCOV332+MPLA: B-COV-S1B-332-526 (1×10⁹)+PBS containing 0.8 mg of MPLA (100 µl)
(5) No administration

For sera from blood collected from the tail vein on Days 0 and 16 from the initiation of the administration, the concentrations of an anti-S1B-RBD protein IgG antibody against the RBD portion contained in the S1B protein were measured by an ELISA method using RayBio^{™} COVID-19 S1 RBD protein Human IgG ELISA Kit (manufactured by RayBiotech).

As a result, it was recognized that the concentration of the anti-S1B-RBD protein IgG antibody was significantly increased in (1), i.e., the serum of the mice to which BCOV332+4 mg of CpG(D35) had been orally administered (FIG. 22B). Thus, it was recognized that the transformed *Bifidobacterium* B-COV-S1B-332-526 had the potential to serve as an active ingredient showing a vaccine effect on a coronavirus infectious disease, especially COVID-19, by mixed administration with CpG D35 as an adjuvant.

### (Example 11) Generation of Bifidobacterium expressing S Protein of Coronavirus on Surface thereof 4

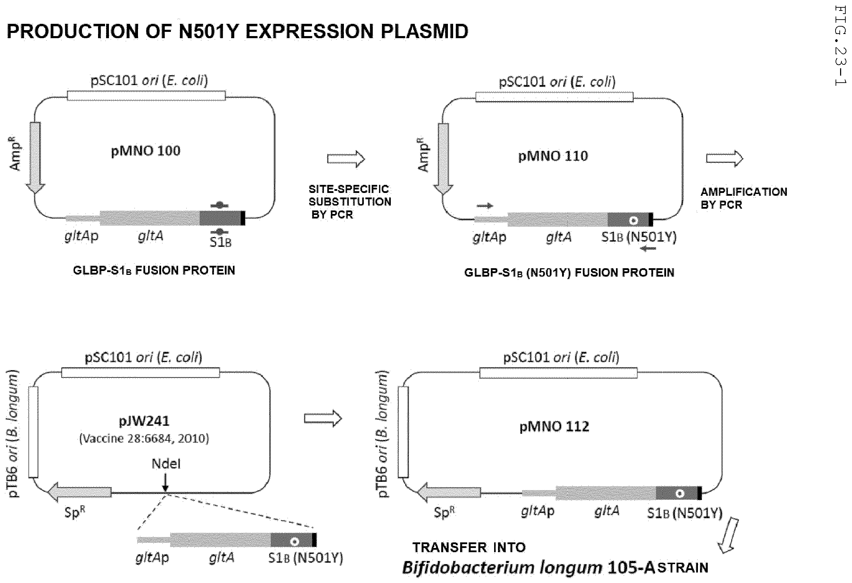

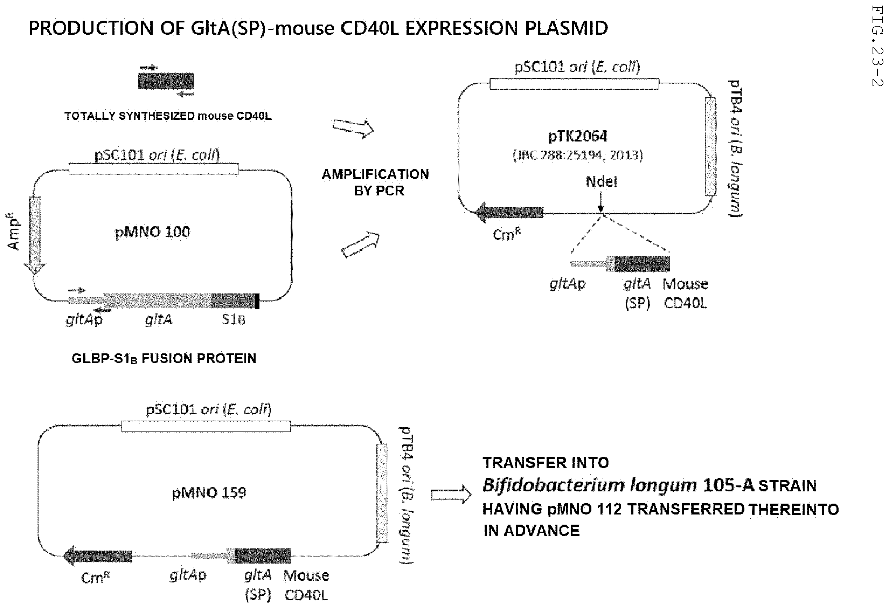

In this Example, the generation of a transformed *Bifidobacterium* B-COV-S1B-332-526 N501Y-CD40L caused to coexpress CD40L having an adjuvant function with the S1B protein (S1B: 332-526 N501Y) is described (see FIG. 23-1 and 23-2).

The generation of a GltA (signal peptide)-mouse CD40L expression plasmid was performed as described below.

First, DNA optimized for codons for the *Bifidobacterium* was totally synthesized (GenScript) on the basis of the amino acid sequence of mouse CD40L. The synthesized sequence is as follows.

· Base sequence of DNA encoding mouse CD40L (SEQ ID NO: 25)

atgatcgaaacctactcccagccgtccccgcgctccgtggccaccggcttgccggcctccatgaagatcttcatgtacctgctgaccgtgttcctg

atcacccagatgatcggctccgtgctgttcgccgtgtacctgcaccgccgcttggacaaggtggaagaagaagtgaacctgcatgaagacttc

gtgttcatcaagaagctgaagcgctgcaacaagggcgaaggctccctgtccctgctgaactgcgaagaaatgcgccgccagttcgaagacct

ggtgaaggacatcaccctgaacaaggaagaaaagaaggaaaactccttcgaaatgcagcgcggcgacgaagacccgcagatcgccgc

ccatgtggtgtccgaagccaactccaacgccgcctccgtgctgcagtgggccaagaagggctactacaccatgaagtccaacctggtgatgct

ggaaaacggcaagcagctgaccgtgaagcgcgaaggcctgtactacgtgtacacccaggtgaccttctgctccaaccgcgaaccgtcctcc

cagcgcccgttcatcgtgggcctgtggctgaagccgtcctccggctccgaacgcatcctgctgaaggccgccaacacccactcctcctcccag

ctgtgcgaacagcagtccgtgcacctgggtggcgtgttcgaactgcaggccggtgcctccgtgttcgtgaacgtgaccgaagcctcccaggtg

atccaccgcgtgggcttctcctccttcggcctgctgaagctgtga

The promoter region and signal peptide (1-28 aa) of gltA of pMNO 100 were amplified using primers formed of base sequences identified by SEQ ID NOS: 26 and 27. Thus, an about 0.5 kbp DNA fragment was obtained.
pTK2064-gltA-f: 5'-GGAAAACTGTCCATACATATGGGCGATGGCGAGG-3' (SEQ ID NO: 26)
pTK2064-gltA-SP-r: 5'-GTCGCTGCCGCAACCGG-3' (SEQ ID NO: 27)

The totally synthesized mouse CD40L gene fragment was amplified using primers formed of base sequences identified by SEQ ID NOS: 28 and 29. Thus, an about 0.8 kbp DNA fragment was obtained.
pTK2064-mCD40L-f: 5'-GGTTGCGGCAGCGACatgatcgaaacctactcccagc-3' (SEQ ID NO: 28)
pTK2064-mCD40L-r: 5'-AGGGCCTCGTGCATAtcacagcttcagcaggccga-3' (SEQ ID NO: 29)

The above-mentioned two kinds of DNA fragments obtained by amplification were inserted into the Ndel site of pTK2064 (Sakurama et al. JBC 288:25194-25206, 2013). In-Fusion HD Cloning Kit (Takara Bio) was used for the insertion. pTK2064 is a plasmid harboring pTB4 ori as the origin of replication for the *Bifidobacterium* and a chloramphenicol resistance gene (CmR) as drug resistance. pTK2064 can coexist with a plasmid having pTB6 ori in the *Bifidobacterium. E. coli* DH5alpha was transformed using the ligation reaction liquid, and the resultant plasmid was named pMNO 159 after the DNA sequence of the full length of gltA (signal peptide)-mouse CD40L (including a promoter) thereon had been determined. The recombinant was selected by chloramphenicol resistance (3 µg/ml). The resultant pMNO 159 was transferred by an electroporation method into a B. *longum 105-A strain* into which the above-mentioned pMNO 112 had been transferred in advance, to generate a recombinant *Bifidobacterium.* The recombinant was selected by spectinomycin resistance (30 µg/ml) and chloramphenicol resistance (3 µg/ml). The resultant transformed *Bifidobacterium* was named B-COV-S1B-332-526 N501Y-CD40L.

### (Example 12) Humoral Immune Response-inducing Effects of Addition of Various Adjuvants

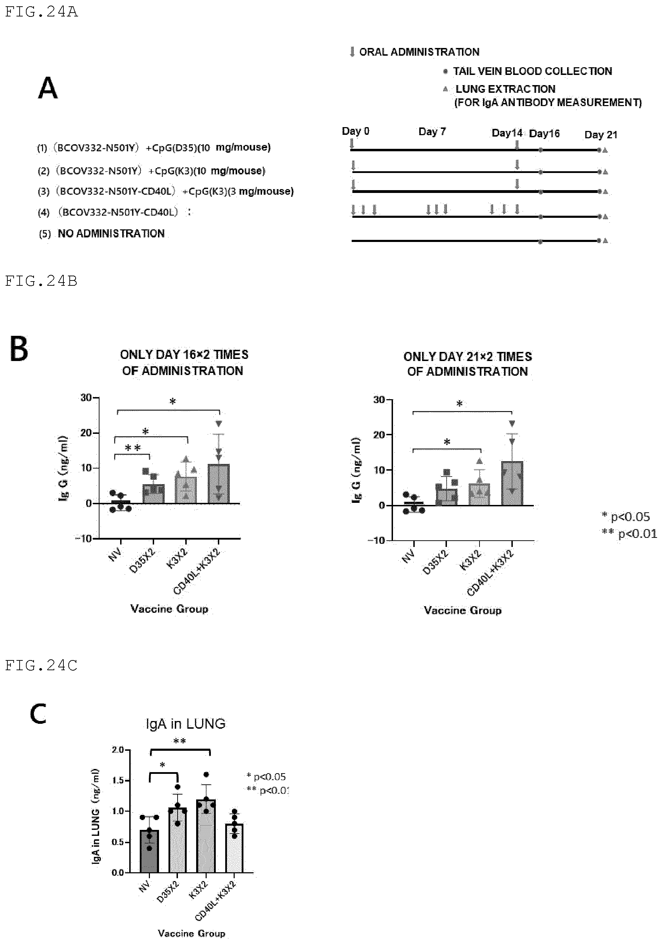

Humoral immune response-inducing effects based on IgG antibody production and IgA antibody production achieved when the transformed *Bifidobacterium* B-COV-S1B-332-526 N501Y generated in Example 3 described above and the transformed *Bifidobacterium* B-COV-S1B-332-526 N501Y-CD40L generated in Example 11 were orally administered to mice (C57BL/6, ♀, 5-week-old, n=5) were determined. The experiments were performed in accordance with an administration protocol illustrated in FIG. 24A in the following systems (1) to (5). Specifically, oral administration was performed on Days 0 and 14 in each of (1) to (3), and oral administration was performed on Days 0, 2, 4, 7, 9, 11, 14, 16, and 18, nine times in total, in (4). (5) was a control with no administration.
(1) (BCOV332-N501Y)+CpG(D35): B-COV-S1B-332-526 N501Y (1×10⁹)+PBS containing 10 mg of CpG(D35) (100 µl)
(2) (BCOV332-N501Y)+CpG(K3): B-COV-S1B-332-526 N501Y (1×10⁹)+PBS containing 10 mg of CpG(K3) (100 µl)
(3) (BCOV332-N501Y-CD40L)+CpG(K3): B-COV-S1B-332-526 N501Y-CD40L (1×10⁹)+PBS containing 3 mg of CpG(K3) (100 µl)
(4) (BCOV332-N501Y-CD40L)×9: B-COV-S1B-332-526 N501Y-CD40L (1×10⁹)+PBS (100 µl)
(5) No administration

### 1. IgG Antibody Production

For sera from blood collected from the tail vein on Days 0, 16, and 21 from the initiation of the administration, IgG amounts were measured by an ELISA method in the same manner as in Example 10. As a result, it was recognized that the concentration of the anti-S1B-RBD protein IgG antibody was increased in each of the sera of the mice to which (1) (BCOV332-N501Y)+CpG(D35), (2) (BCOV332-N501Y)+CpG(K3), and (3) (BCOV332-N501Y-CD40L)+CpG(K3) had been orally administered (FIG. 24B). Thus, it was recognized that the respective transformed *Bifidobacteriums* of B-COV-S1B-332-526 N501Y and B-COV-S1B-332-526 N501Y-CD40L each had the potential to serve as an active ingredient showing a vaccine effect on a coronavirus infectious disease, especially COVID-19, by mixed administration with the CpG adjuvant.

### 2. IgA Antibody Production

Lung tissue harvested on Day 21 from the initiation of administration was homogenized and then suspended with PBS, and the suspension was centrifuged to collect a supernatant. For IgA contained in the supernatant, the concentration of an anti-S1B-RBD protein IgA antibody against the RBD portion contained in the S1B protein was measured by an ELISA method using RayBio^{™} COVID-19 S1 RBD protein Human IgG ELISA Kit (manufactured by RayBiotech). As a result, significant increases in IgA antibody in the lung tissue were able to be recognized in the mice to which (1) (BCOV332-N501Y)+CpG(D35) and (2) BCOV332-N501Y)+CpG(K3) had been orally administered (FIG. 24C). Thus, it was recognized that the transformed *Bifidobacterium* B-COV-S1B-332-526 N501Y had the potential to serve as an active ingredient showing a vaccine effect on a coronavirus infectious disease, especially COVID-19, by oral administration as a mixture with CpG D35 or CpG K3.

### (Example 13) Hamster Infection Experiment 1

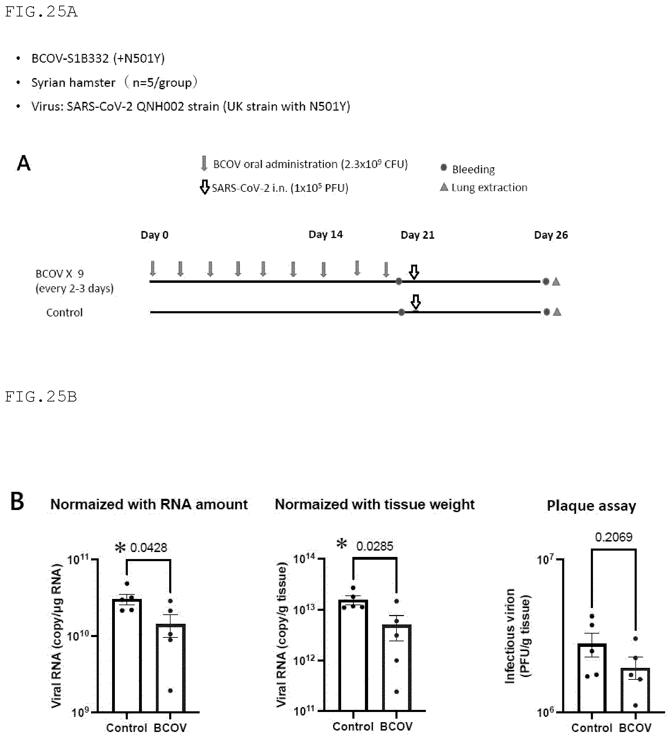

An immune response-inducing effect achieved when the transformed *Bifidobacterium* B-COV-S1B-332-526 N501Y generated in Example 3 described above was orally administered to hamsters (Syrian hamster, ♂, 4-week-old) was determined. A product obtained by mixing B-COV-S1B-332-526 N501Y (2.3×10⁹) into 100 µl of PBS containing an aluminum hydroxide adjuvant (Alum: Alhydrogel^{™} adjuvant 2%, InvivoGen) (BCOV332-N501Y+AI) was orally administered in accordance with an administration protocol with n=5 three times a week over a total of three weeks, nine times in total (FIG. 25A). On Day 21, the SARS-CoV-2 QNH002 strain (UK strain with N501Y) was intranasally administered at 1×10⁵ PFU to cause infection. The hamsters were euthanized on Day 26, and the cardiac blood and lung were harvested.

The results were as follows: the hamsters to which B-COV-S1B-332-526 N501Y and Alum had been orally administered nine times tended to have a small body weight loss after SARS-CoV-2 QNH002 strain (UK strain with N501Y) infection as compared to a no-treatment group, but the difference was not significant. In the lung tissue, significant reductions in viral RNA amount and number of viral plaques in the lung tissue were able to be recognized (FIG. 25B). Thus, it was recognized that B-COV-S1B-332-526 N501Y had the potential to serve as an active ingredient showing a vaccine effect and a severity increase-preventing effect on a coronavirus infectious disease, especially COVID-19 in hamsters as well.

### (Example 14) Hamster Infection Experiment 2

An immune response-inducing effect achieved when the transformed *Bifidobacterium* B-COV-S1B-332-526 N501Y-CD40L caused to coexpress CD40L, which had been generated in Example 11 described above, was orally administered to hamsters (Syrian hamster, ♂, 4-week-old) was determined. A product obtained by mixing B-COV-S1B-332-526 N501Y-CD40L (2.3×10⁹) into 100 µl of PBS (BCOV332-N501Y-CD40L) was orally administered three times a week over a total of three weeks, nine times in total, with n=5 (see FIG. 25A). The same administration protocol as that of Example 13 was carried out, and on Day 21, the SARS-CoV-2 QNH002 strain (UK strain harboring N501Y) was intranasally administered at 1×10⁵ PFU to cause infection. The hamsters were euthanized on Day 26, and their cardiac blood and lungs were harvested.

### 1. Body Weight and Viral Load in Lung Tissue

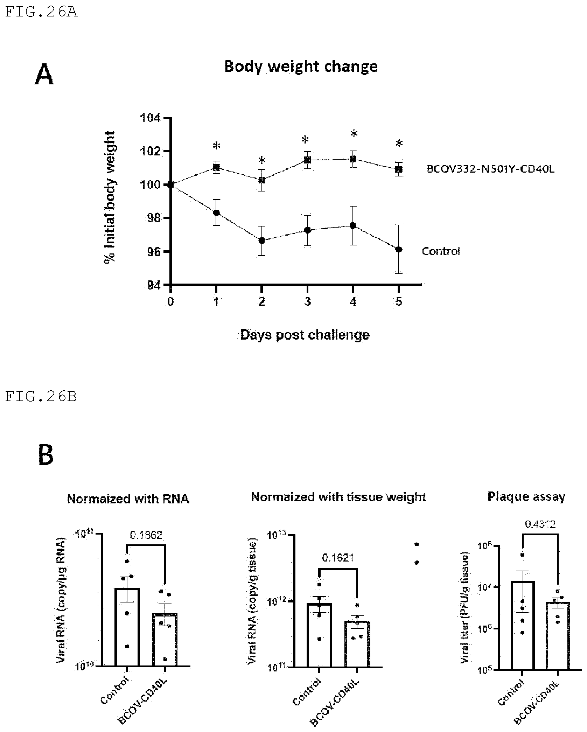

In the BCOV332-N501Y-CD40L administration group, the body weight loss after the infection with the SARS-CoV-2 QNH002 strain (UK strain with N501Y) was significantly suppressed as compared to the control group (no-treatment group) (FIG. 26A). In addition, significant reductions in viral RNA amount and number of viral plaques in the lung tissue were able to be recognized (FIG. 26B).

### 2. Lung and Lung Tissue Findings (Day 26)

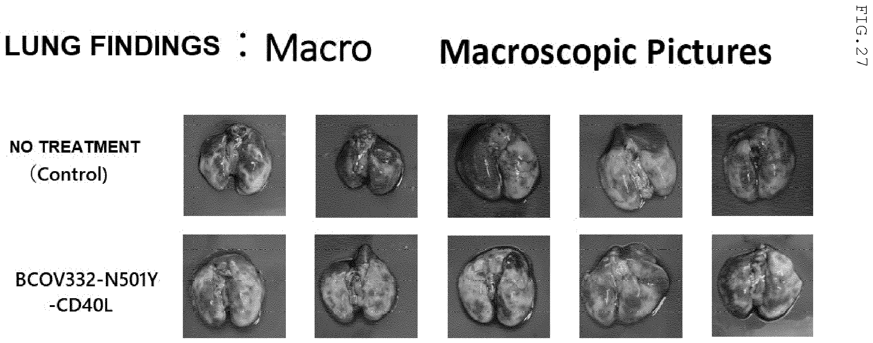

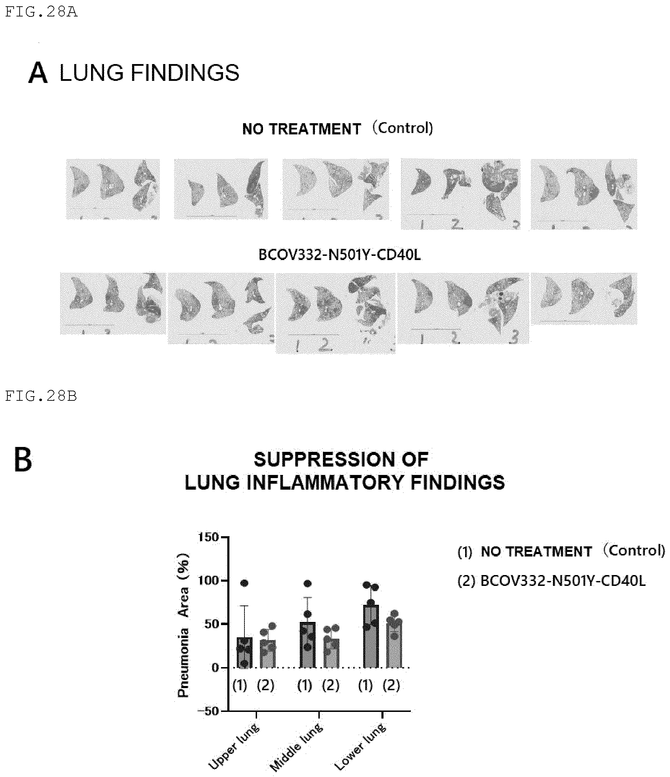

In the BCOV332-N501Y-CD40L administration group, inflammation images of pneumonia were remarkably reduced even after the infection with the SARS-CoV-2 QNH002 strain (UK strain with N501Y) as compared to the control group (no-treatment group) (FIG. 27). In addition, similarly, the lung tissue was subjected to hematoxylin-eosin staining, and the results showed that pneumonia was suppressed with a remarkably small inflammatory region in the BCOV332-N501Y-CD40L administration group as compared to the control group (no-treatment group) at any site of the lung, i.e., the upper lobe (Upper lung), the middle lobe (Middle lung), and the lower lobe (Lower lung) (FIG. 28A and FIG. 28B).

Thus, it was recognized that the transformed *Bifidobacterium* B-COV-S1B-332-526 N501Y-CD40L had the potential to serve as an active ingredient showing a vaccine effect and a severity increase-preventing effect on a coronavirus infectious disease, especially COVID-19 in hamsters as well.

### Industrial Applicability

As described in detail above, the transformed *Bifidobacterium* of the present invention can express and display the part or the whole of the S protein of the coronavirus on the cell surface of the *Bifidobacterium.* By virtue of displaying the part or the whole of the S protein of the coronavirus on the surface of the *Bifidobacterium,* the transformed *Bifidobacterium* can be utilized as an oral vaccine effective for a coronavirus infectious disease, for example, COVID-19. The oral vaccine is easy to ingest even for a child or an elderly person, and besides, is free of pain involved in vaccine inoculation by general injection. In particular, the oral vaccine of the present invention is highly safe by virtue of the use of the *Bifidobacterium,* which has an experience in food. In addition, the transformed *Bifidobacterium* of the present invention can be stored in a freeze-dried form, and hence obviates the need for an oral vaccine formulation to be stored under an ultra-low temperature condition, allowing relatively easy temperature control. Consequently, the oral vaccine formulation of the present invention is easy to handle in transportation and storage, thus showing an extremely useful effect in terms of economy as well.

## Claims

1. A transformed *Bifidobacterium,* which is designed to display a part or a whole of a constituent protein of a coronavirus as a coronavirus antigen on a surface of the *Bifidobacterium.*

2. The transformed *Bifidobacterium* according to claim 1, wherein the part of the constituent protein of the coronavirus is a part or a whole of an S protein of the coronavirus.

3. The transformed *Bifidobacterium* according to claim 1 or 2, wherein the transformed *Bifidobacterium* designed to display on the surface of the *Bifidobacterium* comprises:
DNA encoding the part or the whole of the constituent protein of the coronavirus; and
DNA encoding a membrane protein derived from a bacterium.

4. The transformed *Bifidobacterium* according to claim 3, wherein the membrane protein derived from a bacterium is a GNB/LNB substrate-binding membrane protein derived from a *Bifidobacterium.*

5. The transformed *Bifidobacterium* according to any one of claims 1 to 4, wherein the coronavirus is SARS-COV-2.

6. The transformed *Bifidobacterium* according to claim 5, wherein the S protein of SARS-COV-2 is any one of the following items 1) to 5):
1) a protein identified by an amino acid sequence set forth in SEQ ID NO: 1;
2) a protein identified by an amino acid sequence having one or a plurality of amino acids substituted, deleted, added, or introduced in the amino acid sequence set forth in SEQ ID NO: 1;
3) a protein identified by amino acids of the amino acid sequence set forth in SEQ ID NO: 1 in which amino acid 501 is substituted from asparagine (N) to tyrosine (Y);
4) a protein identified by amino acids of the amino acid sequence set forth in SEQ ID NO: 1 in which amino acid 614 is substituted from aspartic acid (D) to glycine (G); and
5) a protein identified by an amino acid sequence having 60% or more homology to the amino acid sequence set forth in SEQ ID NO: 1.

7. The transformed *Bifidobacterium* according to claim 5 or 6, wherein the part of the S protein of SARS-COV-2 contains an S1 protein.

8. The transformed *Bifidobacterium* according to claim 7, wherein the S1 protein of SARS-COV-2 contains an S1B protein portion of SARS-COV-2.

9. The transformed *Bifidobacterium* according to claim 8, wherein the S1B protein of SARS-COV-2 is any one of the following items 1) to 4):
1) a protein containing at least an amino acid sequence (SEQ ID NO: 2) from position 332 to position 526 of an amino acid sequence set forth in SEQ ID NO: 1;
2) a protein containing an amino acid sequence having one or a plurality of amino acids substituted, deleted, added, or introduced in the amino acid sequence set forth in SEQ ID NO: 2, the protein having immunogenicity for production of an anti-SARS-COV-2 antibody;
3) a protein identified by amino acids of at least the amino acid sequence from position 332 to position 526 of the amino acid sequence set forth in SEQ ID NO: 1 in which amino acid 501 with reference to the amino acid sequence set forth in SEQ ID NO: 1 is substituted from asparagine (N) to tyrosine (Y); and
4) a protein identified by an amino acid sequence having 60% or more homology to the amino acid sequence set forth in SEQ ID NO: 2, the protein having immunogenicity for production of an anti-coronavirus antibody.

10. The transformed *Bifidobacterium* according to any one of claims 4 to 9, wherein the transformed *Bifidobacterium* comprises DNA encoding a protein having an adjuvant function between DNA encoding a part or a whole of an S protein of the coronavirus and DNA encoding a GNB/LNB substrate-binding membrane protein derived from a *Bifidobacterium.*

11. The transformed *Bifidobacterium* according to any one of claims 1 to 10, wherein the coronavirus antigen to be displayed on the surface of the *Bifidobacterium* is a fusion protein of: a protein containing a part or a whole of an S protein of the coronavirus; and a GNB/LNB substrate-binding membrane protein derived from a *Bifidobacterium.*

12. A coronavirus infectious disease vaccine formulation, comprising the transformed *Bifidobacterium* of any one of claims 1 to 11 as an active ingredient of a coronavirus infectious disease vaccine.

13. The coronavirus infectious disease vaccine formulation according to claim 12, wherein the coronavirus infectious disease vaccine formulation is an oral formulation.

14. A coronavirus infectious disease vaccine formulation, comprising as an active ingredient a protein to be displayed on a surface of a *Bifidobacterium,* the protein being produced from the transformed *Bifidobacterium* of any one of claims 1 to 11 and being a part or a whole of a constituent protein of a coronavirus.

15. A method of preventing coronaviral infection and/or a method of preventing an increase in severity after coronaviral infection, comprising administering the coronavirus infectious disease vaccine formulation of any one of claims 12 to 14.

16. A method of preventing and/or treating a sequela after coronaviral infection, comprising administering the coronavirus infectious disease vaccine formulation of any one of claims 12 to 14.

17. A method of boosting immunity against a coronavirus, comprising administering the coronavirus infectious disease vaccine formulation of any one of claims 12 to 14.

18. The method of boosting immunity according to claim 17, wherein the boosting immunity is boosting of humoral immunity and/or cellular immunity.

19. A method of administering a coronavirus infectious disease vaccine formulation, comprising administering the coronavirus infectious disease vaccine formulation of any one of claims 12 to 14 with an adjuvant.

20. A method of administering a coronavirus infectious disease vaccine formulation, comprising administering the coronavirus infectious disease vaccine formulation of any one of claims 12 to 14 by oral administration.

21. A method of generating a coronavirus infectious disease vaccine formulation, comprising a step of performing design so that a part or a whole of a constituent protein of a coronavirus is displayed as a coronavirus antigen on a surface of a *Bifidobacterium.*
